# EUROPÄISCHE PATENTSCHRIFT

(11) **EP 3 079 809 B1**
(45) Veröffentlichungstag und Bekanntmachung des Hinweises auf die Patenterteilung: **02.01.2019**
(21) Anmeldenummer: 14793560.5
(22) Anmeldetag: 05.11.2014
(51) Int. Cl.: C07C 7/13, C10G 25/05, C07C 11/08, B01J 20/06, B01J 20/08, B01J 20/28, C10G 25/00, C10G 53/08, C10G 53/14, C10G 55/04, C10G 57/00, C10G 57/02, C10G 67/06

(54) **REINIGUNG FLÜSSIGER KOHLENWASSERSTOFFSTRÖME MITTELS KUPFERHALTIGER SORPTIONSMITTEL**
CLEANING OF LIQUID HYDROCARBON STREAMS BY MEANS OF COPPER-CONTAINING SORBENTS
NETTOYAGE DE COURANTS D'HYDROCARBURES LIQUIDES AU MOYEN D'UN SORBANT CONTENANT DU CUIVRE

(30) Priorität: 12.12.2013 DE 102013225724
(43) Veröffentlichungstag der Anmeldung: 19.10.2016
(73) Patentinhaber: Evonik Degussa GmbH, 45128 Essen (DE)
(72) Erfinder: GEILEN, Frank, 45721 Haltern am See (DE); PEITZ, Stephan, 45739 Oer-Erkenschwick (DE); STOCHNIOL, Guido, 45721 Haltern am See (DE); WINTERBERG, Markus, 45731 Waltrop (DE); MASCHMEYER, Dietrich, 45657 Recklinghausen (DE); RIX, Armin, 45770 Marl (DE); VOGT, Mathias, 45131 Essen (DE)
(86) Internationale Anmeldenummer: PCT/EP2014/073763
(87) Internationale Veröffentlichungsnummer: WO 2015/086228

(56) Entgegenhaltungen:
- EP-A2- 0 320 979
- WO-A1-94/28089
- US-A1- 2007 034 552
- TURBEVILLE W ET AL: "The chemistry of copper-containing sulfur adsorbents in the presence of mercaptans", CATALYSIS TODAY, ELSEVIER, NL, Bd. 116, Nr. 4, 15. September 2006 (2006-09-15), Seiten 519-525, XP027976148, ISSN: 0920-5861 [gefunden am 2006-09-15]

## Beschreibung

Die Erfindung betrifft ein Verfahren zur Reinigung von Kohlenwasserstoffgemischen, bei welchem ein verunreinigtes Kohlenwasserstoffgemisch enthaltend Kohlenwasserstoffe mit drei bis acht Kohlenstoffatomen durch Kontaktieren mit einem festen Sorptionsmittel zumindest teilweise von Verunreinigungen befreit wird, wobei sich das Kohlenwasserstoffgemisch während des Kontakts mit dem Sorptionsmittel ausschließlich im flüssigen Zustand befindet.

Kohlenwasserstoffe sind Verbindungen, die ausschließlich aus Kohlenstoff und Wasserstoff bestehen. Die Nomenklatur der Kohlenwasserstoffe basiert auf der Anzahl der pro Molekül des Kohlenwasserstoffes enthaltenden Kohlenstoffatome. In Kurzschreibweise wird gern das Präfix Cₙ verwendet, wobei n für die besagte Anzahl steht.

C₄-Kohlenwasserstoffe sind folglich Verbindungen, die ausschließlich aus Kohlenstoff und Wasserstoff bestehen, wobei die Anzahl der Kohlenstoffatome je Molekül vier beträgt. Wichtige Vertreter der C₄-Kohlenwasserstoffe sind die Alkene und Alkane mit vier Kohlenstoffatomen.

Gemische aus C₄-Kohlenwasserstoffen sind Rohstoffe der Petro-Folgechemie. Sie stammen z.B. aus Streamcrackern (so genanntes "Crack C4"), aus katalytischen Crackern (so genanntes "FCC C4" (FCC: "fluid catalytic cracking") oder "DCC C4" (DCC "deep catalytic Cracking), aus Pyrolysen ("Pyrolyse C4"), aus MTO- bzw. MTP-Prozessen (MTO: "methanol to olefins", MTP: methanol to propylene) oder Dehydrierungen von Isobutan und n-Butan. Am verbreitesten sind C₄-Kohlenwasserstoffe aus Streamcrackern (Crack C4) und aus katalytischen Crackern (FCC C4). Es werden auch Gemische von C₄-Gemischen unterschiedlicher Herkunft gehandelt, so genannter "C₄-Schnitt". Zum Zwecke der Verwertung der einzelnen Komponenten sind die C₄-Gemische möglichst sortenrein in ihre Bestandteile zu zerlegen.

Die Aufarbeitung von C₄-Strömen aus Steamcrackern oder katalytischen Crackern wird prinzipiell beschrieben in K.-D. Wiese, F. Nierlich, DGMK-Tagungsbericht 2004-3, ISBN 3-936418-23-3. Eine ausführliche Gesamtprozessbeschreibung findet sich in DE102008007081A1.

Die für diese Erfindung relevanten Aspekte der C₄-Aufarbeitung werden im Folgenden kurz umrissen.

Technische C₄- Kohlenwasserstoffgemische aus den oben beschriebenen Quellen enthalten üblicherweise neben gesättigten und einfach ungesättigten Verbindungen auch mehrfach ungesättigte Verbindungen. Bevor einzelne Verbindungen aus diesen Gemischen isoliert werden können, ist es häufig notwendig, andere Verbindungen möglichst vollständig zu entfernen. Dies kann durch physikalische Methoden, wie z. B. Destillation, Extraktivdestillation oder Extraktion, aber auch durch eine selektive chemische Umsetzung der zu entfernenden Komponenten erfolgen. Besonderes Augenmerk muss dabei auf der möglichst vollständigen Entfernung von den im C₄-Kohlenwasserstoffgemisch enthaltenden Verunreinigungen, wie Sauerstoff-, Stickstoff- und Schwefelhaltigen Komponenten liegen, da diese als Katalysatorgifte negative Auswirkungen auf die einzelnen Prozessschritte haben können. Während diese Verunreinigungen typischerweise in Crack C4 nur in Spuren vorhanden sind, können diese z.B. in FCC C4-Strömen auch in höheren Konzentrationen vorhanden sein.

C₄-Kohlenwasserstoffgemische aus Steamcrackern oder Fluidized Catalytic Crackern weisen typischerweise die in Tabelle 0 aufgeführten Hauptkomponenten auf (Verunreinigungen nicht dargestellt).

**Tabelle 0: Typische Zusammensetzungen von Crack C4 und FCC C4**

| **Komponente** | **Crack C4** | **FCC C4** |
|---|---|---|
| | [Gew.-%] | [Gew.-%] |
| Isobutan | 1 - 3 | 20 - 40 |
| n-Butan | 6 - 11 | 5 - 15 |
| 1-Buten | 14 - 20 | 10 - 20 |
| 2-Butene | 4 - 8 | 20 - 35 |
| Isobuten | 20 - 28 | 10 - 20 |
| 1,3-Butadien | 40 - 45 | kleiner 1 |

Die Zusammensetzung der Rohstoffe kann je nach Herkunft des Materials stark schwanken. Zu den aufgeführten C₄-Komponenten gesellen sich noch Kohlenwasserstoffe mit weniger oder mehr Kohlenstoffatomen, sowie Verunreinigungen wie Mercaptane, Sulfide, Disulfide, Stickstoff- und sauerstoffhaltige Verbindungen in geringen Mengen.

Die Aufarbeitung von FCC C4 kann in einer Variante so erfolgen, dass zunächst die Konzentration des Isobutans mittels eines destillativen Schrittes in einer Destillation auf einen Wert von kleiner 5 Gew.-%, besonders bevorzugt kleiner 3 Gew.-% gesenkt wird. Gleichzeitig werden die im Gemisch vorhandenen Leichtsieder (zum Beispiel C₃-Kohlenwasserstoffe, leichte Sauerstoff, Stickstoff- und Schwefel enthaltende Verbindungen) entfernt bzw. minimiert. Im darauf folgenden Schritt werden in einer Kolonne alle Hochsieder (zum Beispiel C₅-Kohlenwasserstoffe, schwere Sauerstoff-, Stickstoff- und Schwefel enthaltende Verbindungen) über den Sumpf entfernt. Im nächsten Schritt wird Isobuten entfernt, z. B. indem es mit Methanol zu Methyl-tert.-butylether (MTBE) umgesetzt und dieser durch Destillation entfernt wird. Soll reines Isobuten gewonnen werden, kann der Methyl-tert.-butylether anschließend wieder zu Isobuten und Methanol gespalten werden.

Zur weiteren Aufarbeitung des C₄-Gemisches müssen die noch verbliebenen mehrfach ungesättigten Verbindungen mit Hilfe eines Selektivhydrierprozesses zu den entsprechenden einfach ungesättigten und gesättigten Verbindungen umgesetzt werden. Jetzt können 1-Buten und verbliebenes Isobutan in ausreichender Reinheit destillativ abgetrennt und die verbleibenden 2-Butene und das n-Butan weiter aufgearbeitet werden. Häufig werden die 2-Butene durch Oligomerisierung, genauer gesagt, durch Dimerisierung zu Oktenen umgesetzt. Dabei wird aus zwei Molekülen mit je vier Kohlenstoffatomen ein Molekül mit acht Kohlenstoffatome aufgebaut. Die Oktene können anschließend mittels Hydroformylierung zu PVC-Weichmacheralkoholen umgesetzt werden. Die nach Abreaktion der Olefine verbleibenden gesättigten C4-Kohlenwasserstoffe können insbesondere als Treibmittel für Aerosole verwendet werden.

Unter einer Oligomerisierung ist ein Prozess zu verstehen, bei dem aus Olefinen wie insbesondere aus Propen und Butenen, höhere Alkene mit 6-20 Kohlenstoffatomen aufgebaut werden. Industriell angewendet wird beispielsweise der Nickel-katalysierte OCTOL® - Prozess, welcher in Hydrocarbon Process., Int. Ed. (1986) 65 (2. Sect.1), Seiten 31 bis 33 sowie in DE3914817, EP1029839 und DE102004018753 näher beschrieben ist.

Die für die einzelnen Verfahrensschritte verwendeten Einsatzströme haben durch vorangehende Prozesse, in denen immer wieder Verunreinigungen entfernt wurden, in der Regel bereits einen hohen Grad an Reinheit erreicht. Verbliebene Verunreinigungen können jedoch die Katalysatoren reversibel oder auch irreversibel deaktivieren. Diese Deaktivierung soll aus wirtschaftlichen Gründen natürlich auf ein Minimum reduziert werden. Daher sollten so viele Katalysatorgifte wie möglich durch weitere Reinigungsstufen vom Katalysator ferngehalten werden.

Die verschiedenen in den technischen C₄-Gemischen vorhandenen Katalysatorgifte wirken in unterschiedlicher Weise vergiftend. So werden die sauren Katalysatorsysteme oder Systemkomponenten wie Cokatalysatoren fast ausschließlich durch Komponenten vergiftet, die selbst basisch sind oder zumindest durch Folgereaktionen Basen freisetzen. Ein besonders typisches Beispiel für solche Stoffe ist Acetonitril, das als sehr schwache Base vergleichsweise schwer durch Sorptionsprozesse abgetrennt werden kann. Es vergiftet jedoch reversibel starke Lewis-Säuren. In Anwesenheit von Spuren von Wasser hydrolysiert es dort über Acetamid zur starken Base Ammoniak, das dann auch Brönsted-Säuren durch Bildung von Ammoniumionen irreversibel deaktiviert. Ein partielles Katalysatorgift stellt im Übrigen immer auch Wasser selbst dar, dessen Wirkung jedoch in der Regel reversibel ist, sofern es nicht durch weitere Reaktionen zur Bildung stärkerer Katalysatorgifte beiträgt. Für die Nickel-katalysierte Oligomerisierung von Butenen am OCTOL®-Katalysator führt bereits ein Wassergehalt von ca. 5 ppm zu messbarer Deaktivierung. Das Wasser wird jedoch von vielen Systeme an Olefine addiert und die gebildeten Alkohole werden durch die üblichen Katalysatorsysteme über eine Transferhydrierung unter Hydrierung von anderen ungesättigten Komponenten so lange oxidiert, bis das thermodynamische Gleichgewicht erreicht ist.

Auch die Metallkomplexkatalysatoren sind gegen basische Substanzen empfindlich. Die Vergiftungswirkung erfolgt dabei primär zumeist über die Deaktivierung des sauren Cokatalysators.

Die Metallkomponente der Katalysatoren wird hingegen besonders stark durch Komponenten wie Schwefel in Form bestimmter Verbindungen angegriffen, der unter bestimmten Umständen das Metallhydrid oder den Metallkomplex durch Bildung schwerlöslicher Sulfide irreversibel zerstört. Da die Metalle in der Regel in sehr niedrigen Oxidationsstufen vorliegen, sind besonders wirkungsvoll Schwefelverbindungen, die die Metalle zu einer relativ hohen Oxidationsstufe zu oxidieren vermögen, wie zum Beispiel Di- und Polysulfide. Unterschiedliche Schwefelverbindungen vermögen also primär durchaus unterschiedlich zu wirken. Während zum Beispiel Disulfide extrem gut abreagieren zu Thioethern und Schwefel, der dann die Metallhydride unter Bildung von Sulfiden oxidiert, wirken Thioether selbst primär zunächst wohl nur als Lewis-Base. Durch weitere, in der Regel im Detail gar nicht bekannte Prozesse und Reaktionen mit weiteren Spurenkomponenten des Systems führen auch sie aber letztlich auch - wenn auch wesentlich langsamer - zur Bildung von Metallsulfiden.

Nach dem vorangehend gesagten ist also für einen möglichst wirtschaftlichen Betrieb einer Anlage zur Zerlegung von Kohlenwasserstoffgemischen in seine Wertbestandteile mit Hilfe katalytischer Reaktionseinheiten die Aufgabe gestellt, die Katalysatoren möglichst effektiv vor Katalysatorgiften und insbesondere Schwefelverbindungen zu schützen. Dies trifft umso stärker zu, je mehr Edukt der Katalysator spezifisch umsetzen soll, in besonderem Maße also auf heterogene Katalysatoren wie den des OCTOL®-Prozesses.

Schwefelhaltige Giftstoffe werden in den in Frage kommenden Propen- und Butenströmen in der Regel durch eine alkalische Wäsche entfernt. Dabei reagieren Schwefelwasserstoff und Mercaptane besonders gut. In der Regel werden die alkalischen Waschlösungen durch Oxidation mit Luft regeneriert.

Ein solches Waschverfahren wird für den industriellen Einsatz von der Fa. UOP LLC unter dem Namen MEROX® angeboten. (G. A. Dziabis, "UOP MEROX PROCESS" in Robert Meyers, Handbook of Petroleum Refining Processes, 3rd Edition, 2004 McGraw-Hill).

Beim MEROX®-Verfahren werden die Mercaptane in der wässrigen Waschlösung zu Di- und Polysulfiden oxidiert, die als ölige Phase abgetrennt werden. Ein kleiner Teil dieser Di- und Polysulfide verbleibt jedoch in der wässrigen Alkalilauge gelöst oder suspendiert, und es gelingt auch durch eine Wäsche dieser wässrigen Phase mit einem Waschöl oder ähnlichem oft nicht, diesen Rest vor der Rückführung in die Wäsche quantitativ zu entfernen, so dass zwar die Mercaptane weitgehend entfernt, auf der anderen Seite aber kleine Mengen an Di- und Polysulfiden wieder in den Strom eingetragen werden. Diese stellen, wie eben erwähnt, Schwefelkomponenten dar, die die für die Reaktion wesentlichen Metallhydride in schwerlösliche Metallsulfide umwandeln und den Katalysator dadurch irreversibel deaktivieren. Typischerweise enthalten zum Beispiel die Ströme an FCC C4 etwa 100 bis 200 ppm Schwefel. Nach der MEROX®-Wäsche ist dieser Gehalt dann üblicherweise bis auf einen Wert von unter 10 ppm reduziert, wobei die Schwefelverbindungen dann überwiegend aus den genannten Di- und Polysulfiden, aber auch aus höheren Mercaptanen bestehen.

In der Praxis kann man einen Teil der Gifte auch durch geschickte Anordnung von Trennoperationen, wie zum Beispiel Destillationen, in Fraktionen lenken, in denen sie nicht mehr mit empfindlichen Katalysatoren in Berührung kommen. Häufig ist dies aber nicht in dem Umfange möglich, der in Hinblick auf die Reinheit der Ströme wünschenswert erscheint, so dass vor den Katalysatorbetten Sorptionsmittel vorgeschaltet werden müssen, um die erforderliche Reinheit zu gewährleisten.

Sorptionsmittel sind feste Stoffe, die in der Lage sind, einen anderen Stoff, das so genannte Sorbat, an sich zu binden, sofern sie mit dem Sorbat in Kontakt kommen. Die Bindung erfolgt an der Oberfläche des Sorptionsmittels durch physikalische und/oder chemische Effekte. Insoweit unterscheidet man physikalische und chemische Adsorption. Da die Wirkweise eines Sorptionsmittels nicht immer zweifelsfrei feststeht, wird hier wirkungsneutral von einem Sorptionsmittel gesprochen.

Aus technischer Sicht sind Sorptionsmittel allgemein zu unterscheiden in solche, die regenerierbar sind und in solche, die die Katalysatorgifte irreversibel umwandeln bzw. chemisch binden.

Als regenerierbare Sorptionsmittel kommen häufig Molekularsiebe und Zeolithe zum Einsatz. Die regenerierbaren Sorptionsmittel binden Verschmutzungen mit nur moderater Festigkeit. Im Zuge der Regenerierung des Sorptionsmittels werden Bedingungen wie zum Beispiel höheren Temperaturen und niedrigere Drücke eingestellt, unter denen das Sorptionsmittel das Sorbat wieder frei lässt. Diese Eigenschaft führt zu einer relativ geringen Kapazität bis zum Durchbruch. Zusätzlich entstehen oft hohe Betriebskosten durch Freistellen und Spülen des Sorptionsmittels sowie durch die Bereitstellung und Entsorgung der Regeneriergase oder auch der Flüssigkeitsströme.

Irreversible Sorptionsmittel werden dagegen nicht regeneriert sondern nach ihrem Durchbruch entsorgt. Sie müssen daher preiswert verfügbar und entsorgbar sein. Da irreversiblen Sorptionsmittel das Adsorbat chemisch binden, ist ihre Durchlässigkeit gegenüber den zu adsorbierenden Stoffen geringer als bei regenerierbaren Sorptionsmitteln. Irreversible Sorptionsmittel erzielen deswegen bessere Reinheitsgrade als regenerierbare Sorptionsmittel.

EP 0 064 464 A1 beschreibt Kontaktmassen, welche insbesondere zur Entschwefelung von Kohlenwasserstoffchargen verwendbar sind. Die Kontaktmassen enthalten Kupferoxid und basieren auf einem Träger aus Tonerde oder Zeolith von Typ X oder Y. Bedenklich ist der zwingend erforderliche Gehalt an Cadmiumoxid, da Cadmium als kanzerogen eingestuft ist. Kanzerogene Stoffe lassen sich nur aufwendig handhaben und entsorgen, sodass insbesondere der irreversible Einsatz derartiger Kontaktmassen unwirtschaftlich ist.

EP 0 354 316 B1 beschreibt die cadmiumfreie Feinentschwefelung von flüssigen C₄-Kohlenwasserstoffgemischen an Kupfer, Silber und Zink enthaltenden Zeolithen. Der bevorzugte Temperaturbereich liegt zwischen 50 und 130°C, der bevorzugte Druck bei 1 bis 50 bar. Die Raum/Zeit-Belastung wird mit 1 bis 40 h⁻¹ angegeben. Wenngleich das hier beschriebene Sorptionsmittel kein bedenkliches Cadmium enthält, ist dieses Material aufgrund seines hohen Silbergehalts von mindestens 2 Gew.-% ebenso unwirtschaftlich.

Turbeville et al. (W. Turbeville, N. Yap, "The chemistry of copper-containing sulfur adsrobents in the presence of mercaptans", Catalysis Today, Vol. 116 (2006), Seiten 519-525) beschreiben Kupfer-haltige Adsorber zur Entschwefelung von flüssigen Kohlenwasserstoffströmen, insbesondere Naptha. Die Entschwefelung wird dabei bei Temperaturen von 150°C bzw. 190°C durchgeführt.

Besonders anfällig gegen Katalysatorgifte sind Nickel-haltige Oligomerisierungskatalysatoren. Kohlenwasserstoffgemische mit zwei bis vier Kohlenstoffatomen dienen oft als Substrat für Oligomerisierungen wie dem OCTOL®-Prozess. Um Katalysatorgifte effektiv zu entfernen hat sich bewährt, solche Ströme vor dem Eintritt in die Oligomerisierung über ein Molekularsieb zu leiten. So beschreibt etwa EP0395857B1 ein gattungsbildendes Verfahren, bei welchem eine Entschwefelung von Raffineriepropen vor dessen Oligomerisierung an einem mit Kupfer ausgetauschten X-Zeolithen bei einer Temperatur von 120°C, einem Druck von 50 bar abs. und einer Raum/Zeit-Belastung von 0.75 h⁻¹ erfolgt. Bei diesen Bedingungen ist Propen überkritisch.

Da diese einfachen Molekularsiebe leicht verfügbar und gesundheitlich unbedenklich sind, stellen sie in der industriellen Praxis heute das Sorptionsmittel der Wahl zur Feinentschwefelung von C₃ - bis C₈ - Kohlenwasserstoffgemischen dar. Da die Molekularsiebe die Verunreinigungen nur physikalisch binden, lassen sich derartige Sorptionsmittel regenerieren. Allerdings ist ihr Sorptionsvermögen jedoch im Vergleich zu chemisch wirkenden Sorptionsmitteln geringer, sodass mit der Feinentschwefelung an Zeolithen nur mäßige Reinheiten zu erzielen sind.

In Hinblick auf diesen Stand der Technik liegt der Erfindung die Aufgabe zu Grunde, ein Verfahren zur Reinigung von flüssigen C₃- bis C₈-Kohlenwasserstoffgemischen anzugeben, welches auf einem leicht verfügbaren, aber nicht kanzerogenen Sorptionsmittel basiert und welches verglichen mit herkömmlichen Molekularsieben bessere Reinheitsgrade erzielt.

Das Verfahren soll dabei ferner die folgenden Eigenschaften aufweisen:
- Das eingesetzte Sorptionsmittel soll eine möglichst hohe Bindungskapazität für schwefelhaltige Verbindungen aufweisen und diese möglichst vollständig aus dem verunreinigten Kohlenwasserstoffgemisch entfernen;
- Das Verfahren soll geringe Betriebskosten verursachen, insbesondere soll es ohne die permanente Zufuhr zusätzlicher Betriebsstoffe wie beispielsweise Wasserstoff betrieben werden können;
- Das Sorptionsmittel soll ohne eine Vorbehandlung, wie eine Hydrierung oder Oxidierung "out of the box" einsetzbar sein;
- Das Sorptionsmittel soll gefahrlos handhabbar sein, insbesondere soll es keine pyrophoren Eigenschaften zeigen;
- An dem Sorptionsmittel soll es nicht zu Verlust von olefinischen Wertstoffen durch Nebenreaktionen wie Oligomerisierung, Isomerisierung oder Hydrierung kommen.

Gelöst wird diese Aufgabe überraschend dadurch, dass als Sorptionsmittel feste Materialien der folgenden Zusammensetzung verwendet werden:
- Kupferoxid: 10 bis 60 Gew.-% (gerechnet als CuO);
- Zinkoxid: 10 bis 60 Gew.-%, (gerechnet als ZnO);
- Aluminiumoxid: 10 bis 30 Gew.-% (gerechnet als Al₂O₃);
- sonstige Stoffe: 0 bis 5 Gew.-%.

Gegenstand der Erfindung ist mithin ein Verfahren zur Reinigung von Kohlenwasserstoffgemischen, bei welchem ein verunreinigtes, sich ausschließlich im flüssigen Zustand befindliches Kohlenwasserstoffgemisch enthaltend Kohlenwasserstoffe mit drei bis acht Kohlenstoffatomen durch Kontaktieren mit einem festen Sorptionsmittel der folgenden, sich zu 100 Gew.-% ergänzende Zusammensetzung
- Kupferoxid: 10 bis 60 Gew.-% (gerechnet als CuO);
- Zinkoxid: 10 bis 60 Gew.-%, (gerechnet als ZnO);
- Aluminiumoxid: 10 bis 30 Gew.-% (gerechnet als Al₂O₃);
- sonstige Stoffe: 0 bis 5 Gew.-%.
zumindest teilweise von Verunreinigungen befreit wird,
wobei
das verunreinigte Kohlenwasserstoffgemisch mindestens eine Verunreinigung aus einer der folgenden Substanzklassen enthält:
a) Thiole mit der allgemeinen Formel R-SH
   wobei R ein Alkyl-, Aryl-, Cycloalkyl- oder ein Alkenyl-Rest sein kann, wobei es sich bei R insbesondere ein Methyl-, Ethyl-, Propyl-, Butyl-, Phenyl-, Cyclohexyl- oder Butenyl-Rest handelt.
b) Disulfide mit der allgemeinen Formel R-S-S-R',
   wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
c) Sulfide mit der allgemeinen Formel R-S-R'
   wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
d) Substitutierte oder unsubstituierte schwefelhaltige Heterocyclen, insbesondere Thiophene und/oder Thiolane,
dadurch gekennzeichnet,
dass es sich bei den zu entfernenden Verunreinigungen um organische Schwefelverbindungen aus den Substanzklassen a), b), c) und d) handelt,
dass der Kontakt unter den folgenden Bedingungen erfolgt:
- Temperatur zwischen 30°C und 120°C;
- Druck zwischen 0.5 und 3.5 MPa;
- Raum/Zeit-Belastung (weight hour space velocity - WHSV) zwischen 0.5 h⁻¹ und 7 h⁻¹
und dass das verunreinigte Kohlenwasserstoffgemisch eine der folgenden, sich jeweils zu 100 Gew.-% ergänzende Spezifikationen A, B, C oder D erfüllt, wobei sich die angegebenen Gewichtsanteile jeweils auf das Gesamtgewicht des verunreinigten Kohlenwasserstoffgemisches beziehen:
Spezifikation A:
   - Isobutan 20 bis 40 Gew.-%, bevorzugt 30 bis 37 Gew.-%;
   - n-Butan 5 bis 18 Gew.-%, bevorzugt 8 bis 10 Gew.-%;
   - 1-Buten 5 bis 15 Gew.-%, bevorzugt 12 bis 14 Gew.-%;
   - Isobuten 12 bis 25 Gew.-%, bevorzugt 15 bis 20 Gew.-%;
   - 2-Butene 9 bis 40 Gew.-%, bevorzugt 20 bis 30 Gew.-%;
   - 1.3-Butadien 0 bis 3 Gew.-%, bevorzugt 0.5 bis 0.8 Gew.-%;
   - Wasser 0 bis 1 Gew.-%, bevorzugt weniger als 0.1 Gew.-%;
   - Verunreinigungen, insbesondere Schwefel enthaltende Kohlenwasserstoffe von weniger als 0.5 Gew.-%, bevorzugt kleiner 0.2 Gew.-%;
Spezifikation B:
   - Isobutan 0.6 bis 8 Gew.-%, bevorzugt 1 bis 7 Gew.-%;
   - n-Butan 0.5 bis 8 Gew.-%, bevorzugt 4 bis 7 Gew.-%;
   - 1-Buten 9 bis 25 Gew.-%, bevorzugt 10 bis 20 Gew.-%;
   - Isobuten 10 bis 35 Gew.-%, bevorzugt 20 bis 30 Gew.-%;
   - 2-Butene 3 bis 15 Gew.-%, bevorzugt 5 bis 10 Gew.-%;
   - 1.3-Butadien 25 bis 70 Gew.-%, bevorzugt 40 bis 50 Gew.-%;
   - Wasser 0 bis 1 Gew.-%, bevorzugt weniger als 0.5 Gew.-%;
   - Verunreinigungen, insbesondere Schwefel enthaltene Kohlenwasserstoffe weniger als 0.5 Gew.-%, bevorzugt kleiner 0.2 Gew.-%;
Spezifikation C:
   - Isobutan 0.6 bis 8 Gew.-%, bevorzugt 1 bis 7 Gew.-%;
   - n-Butan 0.5 bis 15 Gew.-%, bevorzugt 4 bis 13 Gew.-%;
   - 1-Buten 9 bis 40 Gew.-%, bevorzugt 10 bis 35 Gew.-%;
   - Isobuten 10 bis 55 Gew.-%, bevorzugt 20 bis 50 Gew.-%;
   - 2-Butene 3 bis 25 Gew.-%, bevorzugt 5 bis 20 Gew.-%;
   - 1.3-Butadien 0 bis 1 Gew.-%, bevorzugt weniger als 0.8 Gew.-%;
   - Wasser 0 bis 1 Gew.-%, bevorzugt weniger als 0.5 Gew.-%;
   - Verunreinigungen, insbesondere Schwefel enthaltene Kohlenwasserstoffe weniger als 0.5 Gew.-%, bevorzugt kleiner 0.2 Gew.-%;
Spezifikation D:
   - n-Butan 10 bis 30 Gew.-%, bevorzugt 25 bis 30 Gew.-%;
   - 1-Buten 0.2 bis 45 Gew.-%, bevorzugt 5 bis 30 Gew.-%;
   - 2-Butene 35 bis 85 Gew.-%, bevorzugt 50 bis 75 Gew.-%;
   - Wasser 0 bis 1 Gew.-%, bevorzugt weniger als 0.1 Gew.-%;
   - Verunreinigungen, insbesondere Schwefel enthaltene Kohlenwasserstoffe weniger als 0.5 Gew.-%, bevorzugt kleiner 0.1 Gew.-%;
wobei das Verfahren zur Reinigung von Kohlenwasserstoffgemischen bei einem Wasserstoffgehalt von unter 1 Gew.-ppm bezogen auf die Gesamtmasse des verunreinigten Kohlenwasserstoffgemisches durchgeführt wird.

Die erfindungsgemäß eingesetzten Sorptionsmittel sind in einfacher Weise kommerziell verfügbar, nämlich als Katalysatoren für die Methanolsynthese:
Auf dem Gebiet der Methanolsynthese haben sich Kupfer/Zink/Aluminiumkatalysatoren industriell bewährt. Die Synthese von Methanol erfolgt aus Kohlenmonoxid und Wasserstoff bzw. als Nebenreaktion aus Kohlendioxid und Wasserstoff unter zusätzlichen Erhalt von Wasser. Beide Reaktionen werden also in der Gegenwart von dem Edukt Wasserstoff durchgeführt. Bei Einsatz von Kupfer/Zink/Aluminiumkatalysatoren wird die Methanolsynthese bei Temperaturen zwischen 220°C und 230°C und einem Druck von etwa 5 MPa (50 bar) durchgeführt. Unter diesen Bedingungen liegen die Edukte und Produkte in der Gasphase vor.

Kupfer/Zink/Aluminiumkatalysatoren für die Methanolsythese sind in der Patentliteratur vielfach beschrieben:
So offenbart DE2846614C3 ein Verfahren zur Herstellung von Methanol aus einem Gasgemisch von CO, CO₂ und H₂ bei Temperaturen von 200 bis 350°C in Gegenwart eines Katalysators, der 38.3 % Cu, 48.8 % Zn und 12.9 % Al enthält.

DE1568864C3 weist darauf hin, dass Synthesegas für die Methanolherstellung entschwefelt werden sollte, da sich kupferhaltige Katalysatoren mit Schwefel leicht vergiften lassen. Der hier beschriebene Kupfer/Zink/Aluminiumkatalysator enthält mehr als 35 Gew.-% Kupfer, der Zinkgehalt beträgt 15 bis 50 Gew.-%. Der Aluminiumgehalt wird mit 4 bis 20 Gew. -% angegeben.

EP0125689B2 beschreibt einen Katalysator für die Methanolsynthese, welcher als katalytisch wirksame Substanzen Kupferoxid und Zinkoxid enthält, sowie - als thermostabilisierende Substanz - Aluminiumoxid. Im nicht reduzierten Zustand weisen beispielhaft hergestellte Katalysatorvorstufen etwa 65 bis 68 Gew.-% CuO, 21 bis 23 Gew.-% ZnO und 10 bis 12 Gew.-% Al₂O₃ auf. Die spezifische Oberfläche beträgt 100 bis 130 g/m². Die Methanolsynthese erfolgt bei 250°C und 50 bar.

Ähnliche Methanolkatalysatoren mit 63 bis 65 Gew.-% CuO, 24 bis 27 Gew.-% ZnO und 10 bis 11 Gew.-% Al₂O₃ sind in der DE10160486A1 beschrieben.

Ein Katalysator mit vergleichsweise geringen Kupfer- und hohem Zink-Gehalt (43.2 Gew.-% CuO, 47.0 Gew.-% ZnO und 10.2 Gew.-% Al₂O₃) wurde in US4279781 hergestellt. Dessen katalytische Aktivität in der Methanolsynthese wurde allerdings als eher schlecht bewertet.

Aufgrund der großen industriellen Bedeutung der Synthese der Grundchemikalie Methanol sind Kupfer/Zink/Aluminiumkatalysatoren nicht nur theoretisch in der Patentliteratur beschrieben sondern auch kommerziell leicht verfügbar. Ihre Entsorgung ist vergleichsweise unproblematisch, da keine kanzerogenen Stoffe enthalten sind. Im Übrigen ist das Recycling solcher Sorptionsmittel wirtschaftlich attraktiv, da dieses Material viel wertvolles Kupfer enthält.

Die Erfindung beruht zum Teil auf der Erkenntnis, dass sich kommerziell verfügbare Methanol-Katalysatoren zur Reinigung von typischen Rohstoffströmen der Petro-Folgechemie eignen. Es hat sich nämlich gezeigt, dass derartige Katalysatoren, wenn sie als Sorptionsmittel genutzt, mit flüssigen Kohlenwasserstoffgemischen in Kontakt gebracht werden, auch ohne Zufuhr von Wasserstoff gut mit den Schwefelverbindungen reagieren. Besonders schnell reagieren sie dabei mit Mercaptanen.

Die erfindungsgemäß erkannte Verwendbarkeit von Methanol-Katalysatoren auf CuO/ZnO/Al₂O₃-Basis zur Entgiftung von Kohlenwasserstoffgemischen ist deshalb überraschend, weil die Methanol-Synthese stets in Anwesenheit von Wasserstoff erfolgt, währenddessen Wasserstoff in den zu entgiftenden Stoffströmen in der Regel nicht nennenswert enthalten ist. So sind marktübliche Crack C4- und FCC C4-Ströme frei von Wasserstoff (< 1 Gew.-ppm). Die Entgiftung solcher Ströme erfolgt also quasi in Abwesenheit von Wasserstoff.

Des Weiteren erfolgt die Aufarbeitung von C₃ - bis C₈ -Kohlenwasserstoffgemischen in der Regel in der Flüssigphase, da sich die Kohlenwasserstoffe mit mehr als zwei Kohlenstoffatomen mit geringem Aufwand verflüssigen und dann mit einer hohen Prozessintensität verarbeiten lassen. Die Methanol-Synthese indes erfolgt ausschließlich in der Gasphase. Es war nicht zu erwarten, dass für die Gasphasenkatalyse bestimmten Materialen sich auch zur Flüssigphasensorption eignen würden.

Grundsätzlich eignet sich jeder kommerziell verfügbare Cu/Zn/Al-Katalysator als Sorptionsmittel zur Reinigung der C₃- bis C₈-Kohlenwasserstoffgemische. Bevorzugt werden jedoch solche Katalysatoren eingesetzt, welche die folgende Zusammensetzung aufweisen:
- Kupferoxid: 30 bis 45 Gew.-% (gerechnet als CuO);
- Zinkoxid: 30 bis 50 Gew.-%, (gerechnet als ZnO);
- Aluminiumoxid: 10 bis 15 Gew.-% (gerechnet als Al₂O₃);
- weitere Metalloxide: 0 bis 2 Gew.-%;
- Graphit: 0 bis 3 Gew.-%;
- andere Stoffe: 0 bis 1 Gew.-%.

Als weitere Metalloxide kommen in diesem Zusammenhang beispielsweise Eisenoxide oder Magnesiumoxide in Betracht. Schwermetalloxide, die bekanntermaßen gesundheitsgefährdend sind, wie beispielsweise Cadmium oder Blei oder Chrom, sollte nach Möglichkeit nicht enthalten sein.

Geringere Mengen an Graphit oder Magnesiumstearat dienen als Bindemittel zur besseren Formgebung des Sorptionsmittels. Unter "anderen Stoffen" sind in diesem Zusammenhang produktionsbedingte Verunreinigungen des Sorptionsmittels zu verstehen.

Hinsichtlich der Formgebung kann das Sorptionsmittel in Pulverform oder als Granulat vorliegen. Darüber hinaus kann das Sorptionsmittel in eine makroskopische Form gepresst sein, wie beispielsweise in Kugeln, oder in Pellets oder in Ringe.

Die Herstellung des Sorptionsmittels geeignet sind grundsätzlich alle technischen Methoden, die zu einem Festkörper führen, welcher eine ausreichende Festigkeit zur Handhabung besitzt. Sie umfasst im Wesentlichen die beiden Arbeitsschritte:
y) Bereitstellen eines porösen Gerüstmaterials aus Aluminiumoxid und/oder Graphit;
z) Vermengen des Gerüstmaterials mit Kupferoxid und Zinkoxid;

Eingesetzt werden können Kupferoxidpulver, Kupfercarbonatpulver oder hydroxidhaltige Kupferverbindungen sowie Gemische daraus. Im Falle des Kupfers kann auch eine kupfercarbonathaltige Verbindung mit Hilfe einer ammoniakalischen Lösung ganz oder teilweise in eine Kupfertetramincarbonatlösung überführt werden, die als Einsatzmaterial dient. Diese Substanzen werden entsprechend den erfindungsgemäßen Mischungsverhältnissen zusammen mit Zinkoxid, Zinkcarbonat oder Zinkhydroxid sowie einem Al₂O₃-haltigem Pulver vermischt. Anstelle von Al₂O₃ kann auch teilweise SiO₂ eingesetzt werden. Als Al₂O₃-haltiges Pulver können alle Modifikationen von Al₂O₃ eingesetzt werden sowie auch Aluminiumoxidhydrat oder Aluminiumhydroxyoxide sowie Aluminiumhydroxid. Die einzelnen Feststoffkomponenten können in geeigneten Mischern, Intensivmischern oder Knetern vermengt und homogenisiert werden. Dabei ist es üblich, eine Befeuchtung mit entmineralisiertem Wasser vorzunehmen. Nach ausreichender Mischung kann eine beliebig geeignete Formgebung erfolgen. Unter Umständen ist eine vorhergehende ganz oder teilweise Trockung und/oder Mahlung der Mischung nötig. Für die Formgebung sind beispielsweise Extruder oder Tablettenpressen geeignet. Auch Pelletierteller können für diese Zwecke dienlich sein. Im Falle einer Tablettierung wird dem Gemisch oftmals ein Gleithilfsmittel wie Graphit zugeführt. Im Falle einer Extrusion werden oft andere organische Zusätze gewählt, die geeignet sind die notwendige Plastifizierbarkeit des Gemisches einzustellen. Dazu zählen beispielsweise zelluloseartige Substanzen, Polyether, Polyethylenglycol und andere, die unter Umständen auch als Porenbildner dienen können, wenn die Substanzen durch eine thermische Behandlung, die sich der Formgebung in der Regel anschließt, ganz oder teilweise entfernt werden. Im Falle einer Pelletierung auf einem entsprechenden Pelletierteller wird die Aufbauagglomeration durch die langsame Zugabe einer geeigneten Menge an Wasser erreicht.

Die thermische Behandlung wird in einem Schritt oder in sequentiellen Schritten durchgeführt. Hierbei werden Wasseranteile oder aber organische Anteile entfernt und die mechanische Festigkeit des Formkörpers wird in der Regel dabei erhöht. Außerdem werden die notwendigen Oxidphasen ausgebildet, wenn die Vorstufenmaterialien noch nicht in dieser Form vorlagen.

In einer anderen Art der Herstellung werden Nitratsalze in wässriger Lösung eingesetzt oder die oxydischen Verbindungen werden mit Salpetersäure ganz oder teilweise gelöst. Insbesondere Im Fall der aluminiumoxidischen Verbindungen löst man oftmals nicht vollständig auf sondern modifiziert das Material mit Hilfe der Säure, dieser Vorgang wird als Peptisierung bezeichnet. Das Peptid wird dann mit den anderen gelösten Komponenten wie oben beschreiben vermischt und zu einem Formkörper verarbeitet. Die Temperung führt dann dazu, dass sich aus den Nitraten die jeweiligen Oxide ausbilden können, wenn die Temperatur geeignet gewählt wurde.

Die Verwendung von nitrathaltigen Salzlösungen kann auch dazu führen, dass eine Fällungsreaktion durchgeführt werden muss, um zu einem Feststoffgemisch zu kommen. Die pH-Einstellung erfolgt mit Natronlauge- oder Sodalösungen. Beispiele hierzu finden sich in US4535071.

Weiterhin ist es möglich, Nitratsalzlösungen mittels Sprühtrocknung in ein oxydisches Produktgemisch als Feststoff zu überführen. In der Regel folgen dann eine Mahlung und eine wie zuvor beschriebene Formgebung. Eine abschließende Temperung, die aber auch direkt nach der Sprühtrockung oder der Mahlung der Bestandteile durchgeführt werden kann, führt die notwendige restliche Nitratzersetzung herbei und überführt die Komponenten hinzu den Oxiden und verfestigt den Formköper.

Die vorstehend beschriebene Eigenfertigung des Sorptionsmittels kann durch Verwendung eines kommerziell verfügbaren Methanol-Katalysators entfallen. Geeignet sind beispielsweise MegaMax® 700 und 800 von Clariant (vormals Süd-Chemie) und Haldor Topsoe's Mk-101 und Mk-121. Beschrieben sind diese Katalysatoren in Nitrogen + Syngas 290, November-December 2007, Seite 36.

Im Gegensatz zur Methanol-Synthese wird das erfindungsgemäße Reinigungsverfahren in Abwesenheit von Wasserstoff durchgeführt. Eine 100%ige Abwesenheit von Wasserstoff lässt sich in der industriellen Technik natürlich nicht gewährleisten. Unter der "Abwesenheit von Wasserstoff" soll deswegen ein Wasserstoffgehalt von unter 1 Gew-ppm bezogen auf die Gesamtmasse des verunreinigten Kohlenwasserstoffgemisches verstanden werden.

Das Sorptionsmittel wird vorzugsweise als Reinigungsbett direkt vor dem zu schützten Katalysator aufgeschüttet. Es kann sich im demselben Gefäß befinden wie der zu schützende Katalysator (also im Reaktor) oder in einem separat davor angeordneten Gefäß. Die Anordnung des Reinigungsbetts im Reaktor ist deshalb möglich, weil dem Sorptionsmittel keine Reaktionswärme ab- oder zugeführt werden muss. Je nach den Umständen werden üblicherweise Verweilzeiten zwischen 0.01 und 0.2 Stunden im Reinigungsbett vorgesehen, bei Bedarf aber auch darüber hinaus. Da der Betrieb bei erhöhter Temperatur die Abreaktion beschleunigt und die Schwefelkapazität erhöht, ist es vorteilhaft, sie nach den meist vorhandenen Vorwärmern anzuordnen. Die Einhaltung einer bestimmten Temperatur des Sorptionsmittels ist maßgeblich für sein Reinigungsvermögen. Versuche zeigen, dass der Kontakt deswegen bei Temperaturen zwischen 30 °C und 120 °C stattfinden soll. Die optimale Kontakttemperatur liegt bei ca. 80°C. Da kommerzielle Methanol-Katalysatoren bei deutlich höheren Temperaturen eingesetzt werden, ist Temperaturstabilität in diesen Bereichen gegeben. Falls der zu schützende Katalysator bei einer anderen Temperatur betrieben wird, sollte das Sorptionsmittel in einem separaten Gefäß, also außerhalb des Reaktors angeordnet werden.

Wichtig ist, dass sich das verunreinigte Kohlenwasserstoffgemisch während des Kontakts mit dem Sorptionsmittel ausschließlich im flüssigen Zustand befindet. Im angegebenen Temperaturbereich wird dies durch einen Druck zwischen 0.5 und 3.5 MPa (5 bis 35 bar) gewährleistet. Letztendlich kommt es auf den Druck jedoch nicht an, solange sich die Kohlenwasserstoffe im flüssigen Zustand befinden. Die Raum-/Zeit-Belastung (weight hour space velocity - WHSV) wird dann zwischen 0.5 und 7 h⁻¹ gewählt. Dies bedeutet, dass pro Kilogramm Sorptionsmittel zwischen 0.5 und 7 Kilogramm pro Stunde verunreinigtes Kohlenwasserstoffgemisch über das Reinigungsbett gefahren wird. Das Reinigungsbett besteht aus einer Schüttung des Sorptionsmittels mit einer Schüttdichte im Bereich von 0.7 bis 1.5 kg/m³, bevorzugt etwa 1.15 kg/m³.

Das Sorptionsmittel wird üblicherweise in einem oxidierten Zustand angeliefert, der eine Handhabung bei Raumtemperatur an der Luft zulässt. Nach dem Befüllen der Reaktoren müssen die Sorptionsmittel nicht mehr durch eine Nachreduktion aktiviert werden. Auch nach dem Einsatz müssen die Sorptionsmittel nicht durch Oxidation mit Luft stabilisiert werden, so dass sie in einfacher Weise aus dem Reaktor entnommen werden können.

Um eine besonders effektive Reinigung zu erreichen und Betriebsunterbrechungen durch Wechsel des Sorptionsmittels zu vermeiden, empfiehlt es sich, mehrere Behälter einzusetzen, die derart revolvierend in Reihe geschaltet werden können, dass am Eintritt immer der Behälter mit der höchsten und zu Austritt hin immer der mit der niedrigsten Beladung angeordnet ist. Mindestens ein Behälter kann dabei, ohne den zu reinigenden Strom zu unterbrechen, herausgenommen und das in ihm befindliche Material gespült und entnommen werden, danach erfolgt in analoger Weise die Neubefüllung.

Die Verwendung von Material mit einer großen Kupferoxidoberfläche ist vorteilhaft, weil die Reaktionsgeschwindigkeit der Adsorption und der Umwandlung mit ihr korreliert und diese Materialien auch eine höhere Sorptionskapazität aufweisen. Bevorzugt weist das Sorptionsmittel eine Kupferoxidoberfläche von mindestens 50 m²/g, bevorzugt 100 m²/g, bezogen auf seinen Kupferoxidgehalt auf. Dies begünstigt die Sorptionswirkung. Die Oberfläche wird per Stickstoff-Sorption bestimmt.

Wichtig im Sinne der vorliegenden Erfindung ist, dass das Sorptionsmittel keine wesentliche katalytische Aktivität zur Hydrierung, Veretherung, Oligomerisierung oder weiteren Reaktionen von Olefinen aufweist. Die genannten Reaktionen der Kohlenwasserstoffe sollen ausschließlich an den dafür vorgesehenen Katalysatoren, nicht aber im Reinigungsbett erfolgen. Die zu schützenden Katalysatoren befinden sich also außerhalb des Reinigungsbettes, zumindest in einer anderen Schüttung oder in anderen Apparaten.

Das erfindungsgemäße Verfahren eignet sich für die Aufreinigung aller Kohlenwasserstoffgemische, bei welchem ein verunreinigtes Kohlenwasserstoffgemisch mit drei bis acht Kohlenstoffatomen enthalten wird. Als technisch relevant sind davon zu erachten z.B. Propen, n-Butene, n-Pentene, Hexene, Neohexen etc sowie deren gesättigte Analoga. Von ihnen nehmen Propan/Propen und die Butane/Butene die absolut bedeutendste Stellung ein.

Das erfindungsgemäße Sorptionsmittel lässt sich besonders vorteilhaft einsetzen zum Reinigen von typischen C₄-Kohlenwasserstoffströme in einem Aufarbeitungszustand unmittelbar vor Umsetzung der darin enthaltenen Butene. Die "Verunreinigungen" umfasst neben den Schwefel enthaltenden Verbindungen auch Basen wie beispielsweise Amine oder Nitrile, die allerdings unter der Nachweisgrenze liegen.

Das Verfahren ist auf solche Gemische besonders gut anwendbar, da es als Gifte für die heterogenen, aluminium-, silicium- oder nickelhaltigen Oligomerisierungskatalysatoren wirkenden Verunreinigungen gut entfernt.

Bei den Verunreinigungen, die erfindungsgemäß aus dem verunreinigten Kohlenwasserstoffgemisch entfernt werden sollen, handelt es sich um organische Schwefelverbindungen, die in der nachfolgenden Aufarbeitung des Kohlenwasserstoffgemisches als Katalysatorgift wirken. Zu katalysatorschädlichen, organischen Schwefelverbindungen, die in den üblicherweise erhältlichen Rohstoffströmen enthalten sind, gehören insbesondere:
a) Thiole mit der allgemeinen Formel R-SH,
b) Disulfide mit der allgemeinen Formel R-S-S-R',
c) Sulfide mit der allgemeinen Formel R-S-R' und
d) substituierte oder unsubstituierte schwefelhaltige Heterozyklen, wie insbesondere Thiophene und/oder Thiolane.

In den oben angegebenen Strukturformeln können R und R' gleiche oder unterschiedliche Alkyl-, Aryl-, Cycloalkyl-, oder Alkenyl-Reste sein, wobei es sich bei R und R' insbesondere um Methyl-, Ethyl-, Propyl-, Butyl-, Phenyl-, Cyclohexyl- oder Butenyl-Reste handelt.

Der besondere Vorteil des erfindungsgemäß eingesetzten Sorptionsmaterials besteht darin, dass es die Verunreinigungen chemisch adsorbiert und zwar insbesondere dadurch, dass als Verunreinigung enthaltene Thiole an der Oberfläche des Sorptionsmittels arretiert werden. Etwaige Disulfide werden an dem Sorptionsmittel in ein Thiol umgesetzt und dann arretiert. Die Chemiesorption bewirkt einen besonders hohen Reinigungsgrad, sodass das Kohlenwasserstoffgemisch nahezu vollständig von enthaltenen Thiolen und Disulfiden befreit wird.

Die Chemisorption der Katalysatorgifte ist irreversibel. Aus diesem Grunde kann das erfindungsgemäß eingesetzte Sorptionsmittel nicht regeneriert werden. Dies bedeutet, dass stark verunreinigte Kohlenwasserstoffströme das Sorptionsmittel rasch erschöpfen, sodass es ausgetauscht werden muss. Im Interesse des wirtschaftlichen Betriebs des Reinigungsverfahrens sollte der Gewichtsanteil der Verunreinigungen in dem verunreinigten Kohlenwasserstoffgemisch bezogen auf dessen Gesamtgewicht kleiner als 0.2 Gew.-% sein. Besonders bevorzugt enthält das verunreinigte Kohlenwasserstoffgemisch weniger als 100 Gew.-ppm und besonders bevorzugt weniger als 10 Gew.-ppm Verunreinigungen, jeweils gerechnet als Schwefel. Bei einem solch geringen Verunreinigungsgrad lässt sich das Sorptionsmittel sehr lange betreiben und ermöglicht zudem eine nahezu restlose Entfernung der Katalysatorgifte.

Nun weisen die üblichen aus Erdölraffinerien stammenden Rohstoffgemische Schwefelgehalte von weit über 0.2 Gew.-% auf. Aus diesem Grunde ist es erforderlich, das Rohstoffgemisch in einer der sorptiven Reinigung vorgeschalteten Vorreinigungsstufe vorzureinigen. In der Vorreinigungsstufe wird das stärker verunreinigte Rohstoffgemisch unter Erhalt eines Kohlenwasserstoffgemisches vorgereinigt, dessen Verunreinigungsgrad unter 0.2 Gew.-% liegt.

Als Vorreinigungsstufe eignet sich insbesondere die oben beschriebene MEROX®-Wäsche oder eine Thioveretherung, wie sie in der zum Anmeldezeitpunkt noch unveröffentlichten DE102012212317A1 offenbart ist.

Die erfindungsmäße Form der Reinigung eignet sich insbesondere dafür, hinter einer MEROX®-Wäsche als Polizei-Filter in den Strom eingeschaltet zu werden.

Unter einem Polizei-Filter ist in diesem Zusammenhang eine zweite Reinigungsinstanz zu verstehen, die hinter einer ersten Reinigungsinstanz angeordnet ist und deren Funktion darin besteht, von der ersten Reinigungsinstanz nicht erfasste Restmengen der Katalysatorgifte endgültig von nachfolgenden Reaktionsschritten fernzuhalten oder im Falle einer Betriebsstörung in der ersten Instanz eine sofortige Schädigung der nachfolgenden Reaktionsschritte auszuschließen.

Als erste Reinigungsinstanz dient vorzugsweise eine MEROX®-Wäsche, welche die meisten Katalysatorgifte in größeren Mengen vorweg abscheidet. Lediglich die von der MEROX®-Wäsche nicht erfassten Mercaptane und Disulfide werden dann erfindungsgemäß im Sorptionsbett zurückgehalten. Für den Fall einer Betriebsstörung in der MEROX®-Anlage übernimmt das Sorptionsmittel deren volle Reinigungsfunktion und schützt die Oligomerisierung vor sofortigen irreversiblen Schäden. Da der Polizei-Filter im normalen Betriebzustand nur eine geringe Menge Adsorbat aufnimmt, darf er kapazitiv deutlich kleiner ausgelegt werden als eine MEROX® -Wäsche. Entsprechend rasch ist er bei einem Störfall erschöpft. Die geeignete Dimensionierung des Polizei-Filters hängt davon ab, wie schnell das anströmende Gemisch umgeleitet werden kann.

Thioether als vergleichsweise unreaktive Substanzen werden in MEROX®-Wäschen praktisch nicht entfernt. Um allzu große Konzentrationen am Eintritt in das Sorptionsbett zu vermeiden, werden sie vorzugsweise an einer geeigneten Stelle im Prozessablauf vor dem Sorptionsbett als Hochsieder in einer Destillation abgetrennt.

In Kombination mit einer Vorreinigungsstufe wie einer MEROX®-Wäsche kann das hier beschriebene Sorptionsmittel bedenkenlos irreversibel eingesetzt werden. Unter einem irreversiblen Einsatz ist in diesem Zusammenhang zu verstehen, dass keine unmittelbare Regeneration, also Wiedergewinnung des aktiven Sorptionsmittels erfolgt, sobald dieses desaktiviert ist. Dies schließt nicht aus, dass das verbrauchte Sorptionsmittel recycelt wird, indem die darin enthaltenen Metalle, wie insbesondere das Kupfer, metallurgisch wiedergewonnen werden. Bei einer solchen metallurgischen Behandlung geht nämlich die ursprüngliche Zusammensetzung des Sorptionsmittels verloren, sodass in diesem Zusammenhang nicht von einer Regeneration gesprochen werden kann.

Das erfindungsgemäße Verfahren eignet sich grundsätzlich zur Entschwefelung von Kohlenwasserstoffströmen mit drei bis acht Kohlenstoffatomen. Hier wird es jedoch zur Entgiftung von C₄-Strömen verwendet, die als Crack C4 oder als FCC C4 oder ihre entsprechenden Raffinate bei der Raffinierung von Erdöl anfallen. Das verunreinigte Kohlenwasserstoffgemisch erfüllt eine der folgenden, sich jeweils zu 100 Gew.-% ergänzenden Spezifikationen A, B, C oder D, wobei sich die angegebenen Gewichtsanteile jeweils auf das Gesamtgewicht des verunreinigten Kohlenwasserstoffgemisches beziehen.
Spezifikation A:
   - Isobutan 20 bis 40 Gew.-%, bevorzugt 30 bis 37 Gew.-%;
   - n-Butan 5 bis 18 Gew.-%, bevorzugt 8 bis 10 Gew.-%;
   - 1-Buten 5 bis 15 Gew.-%, bevorzugt 12 bis 14 Gew.-%;
   - Isobuten 12 bis 25 Gew.-%, bevorzugt 15 bis 20 Gew.-%;
   - 2-Butene 9 bis 40 Gew.-%, bevorzugt 20 bis 30 Gew.-%;
   - 1.3-Butadien 0 bis 3 Gew.-%, bevorzugt 0.5 bis 0.8 Gew.-%;
   - Wasser 0 bis 1 Gew.-%, bevorzugt weniger als 0.1 Gew.-%;
   - Verunreinigungen, insbesondere Schwefel enthaltende Kohlenwasserstoffe von weniger als 0.5 Gew.-%, bevorzugt kleiner 0.2 Gew.-%;
Spezifikation B:
   - Isobutan 0.6 bis 8 Gew.-%, bevorzugt 1 bis 7 Gew.-%;
   - n-Butan 0.5 bis 8 Gew.-%, bevorzugt 4 bis 7 Gew.-%;
   - 1-Buten 9 bis 25 Gew.-%, bevorzugt 10 bis 20 Gew.-%;
   - Isobuten 10 bis 35 Gew.-%, bevorzugt 20 bis 30 Gew.-%;
   - 2-Butene 3 bis 15 Gew.-%, bevorzugt 5 bis 10 Gew.-%;
   - 1.3-Butadien 25 bis 70 Gew.-%, bevorzugt 40 bis 50 Gew.-%;
   - Wasser 0 bis 1 Gew.-%, bevorzugt weniger als 0.5 Gew.-%;
   - Verunreinigungen, insbesondere Schwefel enthaltene Kohlenwasserstoffe weniger als 0.5 Gew.-%, bevorzugt kleiner 0.2 Gew.-%.
Spezifikation C:
   - Isobutan 0.6 bis 8 Gew.-%, bevorzugt 1 bis 7 Gew.-%;
   - n-Butan 0.5 bis 15 Gew.-%, bevorzugt 4 bis 13 Gew.-%;
   - 1-Buten 9 bis 40 Gew.-%, bevorzugt 10 bis 35 Gew.-%;
   - Isobuten 10 bis 55 Gew.-%, bevorzugt 20 bis 50 Gew.-%;
   - 2-Butene 3 bis 25 Gew.-%, bevorzugt 5 bis 20 Gew.-%;
   - 1.3-Butadien 0 bis 1 Gew.-%, bevorzugt weniger als 0.8 Gew.-%;
   - Wasser 0 bis 1 Gew.-%, bevorzugt weniger als 0.5 Gew.-%;
   - Verunreinigungen, insbesondere Schwefel enthaltene Kohlenwasserstoffe weniger als 0.5 Gew.-%, bevorzugt kleiner 0.2 Gew.-%.
Spezifikation D:
   - n-Butan 10 bis 30 Gew.-%, bevorzugt 25 bis 30 Gew.-%;
   - 1-Buten 0.2 bis 45 Gew.-%, bevorzugt 5 bis 30 Gew.-%;
   - 2-Butene 35 bis 85 Gew.-%, bevorzugt 50 bis 75 Gew.-%;
   - Wasser 0 bis 1 Gew.-%, bevorzugt weniger als 0.1 Gew.-%;
   - Verunreinigungen, insbesondere Schwefel enthaltene Kohlenwasserstoffe weniger als 0.5 Gew.-%, bevorzugt kleiner 0.1 Gew.-%.

Die Spezifikation A beschreibt dabei typisches FCC C4, währenddessen die Spezifikation B typisches Crack C4 beschreibt. Spezifikation C beschreibt ein typisches Raffinat I aus Crack C4. Spezifikation D schließlich beschreibt ein Raffinat III aus FCC oder CC4.

Nachdem das verunreinigte Kohlenwasserstoffgemisch erfindungsgemäß von seinen Katalysatorgiften befreit wurde, kann die übliche Aufarbeitung solcher Gemische erfolgen, ohne dabei eine Vergiftung der stromabwärts eingesetzten Katalysatoren befürchten zu müssen. Zu den typischen Aufarbeitungsschritten, die auf die hier beschriebene Reinigung folgen können, gehören:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.

Selbstverständlich müssen nicht alle aufgezählten Aufarbeitungsschritte a) bis i) durchgeführt werden, es können auch nur einzelne durchgeführt werden. Auch ist die aufgezählte Reihenfolge nicht bindend.

Darüber hinaus können einzelne der aufgezählten Aufarbeitungsschritte auch vor der erfindungsgemäßen Reinigung angeordnet sein, sofern diese nicht gegen die Katalysatorgifte empfindlich sind. Zumindest eine Nickel-katalysierte Oligomerisierung sollte mit dem erfindungsgemäßen Sorptionsmittel geschützt werden, da organische Schwefelverbindungen selbst in geringsten Konzentrationen Nickel-Katalysatoren vergiften.

Sofern das eingesetzte Kohlenwasserstoffgemisch auch mit Wasser verunreinigt ist, empfiehlt es sich das mit Wasser verunreinigte Kohlenwasserstoffgemisch vor Eintritt in das Reinigungsbett von Wasser zu befreien, also zu trocknen. Die Abtrennung des Wassers geschieht aus folgender Motivation: Da homogen gelöstes Wasser im Gemisch die Wirkung des Sorptionsmittels etwas abschwächt, wird der Strom vorzugsweise vor Eintritt in das Reinigungsbett getrocknet, beispielsweise durch eine Azeotropdestillation (Trocknungsdestillation).

Den prinzipiellen Aufbau solcher Verwertungsketten unter Einbindung der erfindungsgemäßen Entgiftung sollen im Folgenden näher dargestellt werden. Hierfür zeigen schematisch:
- Figur 1:: C₄-Strang mit eingangs angeordneter Grob- und Feinentschwefelung;
- Figur 2:: C₄-Strang mit sorptiver Reinigung unmittelbar vor der Oligomerisierung.

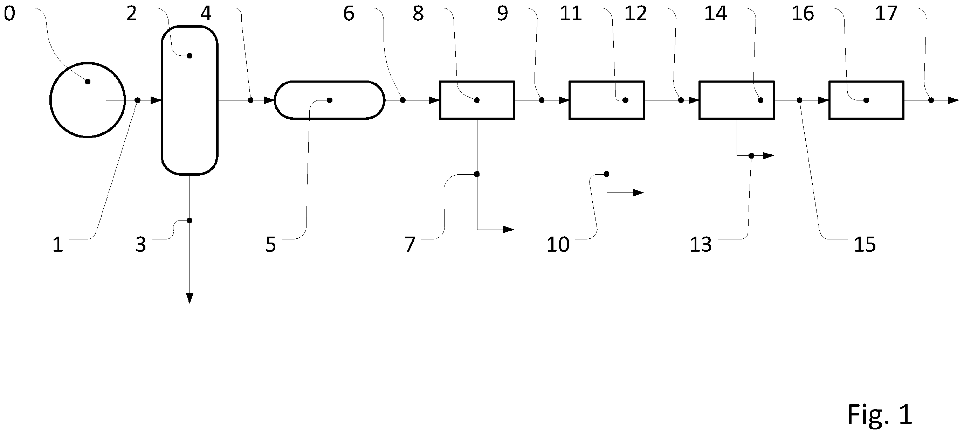

Figur 1 zeigt schematisch einen Strang zur Aufarbeitung von C₄-Kohlenwasserstoffgemischen.

Eine Rohstoffquelle 0 liefert ein Rohstoffgemisch 1, welches überwiegend Kohlenwasserstoffe mit vier Kohlenstoffatomen (Butene und Butane) enthält. Bei der Rohstoffquelle 0 kann es sich beispielsweise um eine Erdöl-Raffinerie handeln. Je nachdem, ob der Cracker fluidkatalytisch arbeitet oder als Dampfcracker betrieben wird, wird ein daraus erhaltenes Rohstoffgemisch 1 als FCC C4 oder als Crack C4 bezeichnet.

Als alternative Rohstoffquellen 0 bzw. Rohstoffgemische 1 kommen auch DCC C4 (DCC: "Deep catalytic cracking"), Pyrolyse C4, C4 aus MTO ("methanol-to-olefins") bzw. MTP ("methanol-to-propylene")-Prozessen oder C₄ aus Dehydrierungen von n-Butan in Betracht.

Da rohe C₄-Ströme abhängig von ihrer Quelle 0 stark schwefelhaltig sein können, wird das Rohstoffgemisch 1 zunächst in einer Vorreinigungsstufe 2 grob vorgereinigt, indem schwefelhaltige Bestandteile 3 in größeren Mengen abgetrennt werden. Bei der Vorreinigungsstufe 2 kann es sich beispielsweise um eine MEROX®-Wäsche oder um eine Thioveretherung handeln. Alternativ kann hier auch ein reversibles Sorptionsmittel eingesetzt werden, welches zyklisch regeneriert wird. Da die Abtrennungsleistung einer MEROX®-Wäsche oder einer Thioveretherung jedoch deutlich größer ist, sind diese Vorreinigungsstufen einer sorptiven Grobreinigung vorzuziehen.

Aus der Vorreinigungsstufe 2 wird dann ein Kohlenwasserstoffgemisch 4 abgezogen, welches immer noch verunreinigt ist (Verunreinigungsgrad max. 0.2 Gew.-%, bevorzugt unter 100 Gew.-ppm). Das verunreinigte Kohlenwasserstoffgemisch 4 wird zur vollständigen Beseitigung der darin enthaltenen Katalysatorgifte in ein Reinigungsbett 5 gefahren. Bei dem Reinigungsbett 5 handelt es sich um eine Schüttung aus einem Kupferoxid, Zinkoxid und Aluminiumoxid enthaltenen Feststoff, dem Sorptionsmittel. Das Reinigungsbett 5 ist in einem an sich bekannten Gefäß enthalten. Das Gefäß wird von dem flüssigen, verunreinigten Kohlenwasserstoffgemisch 4 durchflossen, sodass das darin enthaltene Sorptionsmittel die in dem Kohlenwasserstoffgemisch 4 enthaltenen Verunreinigungen chemisch adsorbiert und damit in dem Reinigungsbett 5 arretiert. Auf diese Weise wird ein gereinigtes Kohlenwasserstoffgemisch 6 erhalten, welches nahezu vollständig von Katalysatorgiften befreit ist.

Entsprechend seiner Wertstoffzusammensetzung erfolgt nun eine an sich bekannte Aufarbeitung der im Rohstoffgemisch 1 enthaltenen Wertstoffe. Sofern es sich bei dem Rohstoffgemisch 1 um Crack C4 handelt, weist dieses einen hohen Gehalt an Butadien 7 auf, welches in einer Butadien-Abtrennung 8 extraktiv entfernt wird.

Reste des nicht extrahierten Butadiens werden selektiv hydriert (nicht dargestellt). Man erhält sogenanntes "Raffinat I" 9.

Das im Raffinat I enthaltene Isobuten 10 wird in einer Isobuten-Abtrennung 11 entfernt. Die Isobuten-Abtrennung 10 besteht in der Regel aus einer MTBE-Synthese, in welcher das Isobuten mit Methanol zu Methyl-tert.-Butylether (MTBE) umgesetzt wird und einer nachgelagerten MTBE-Spaltung, in welcher das MTBE in Isobuten 10 zurückgespalten wird.

Das von Isobuten befreite Gemisch wird als "Raffinat II" 12 bezeichnet. Der darin enthaltene Wertstoff 1-Buten 13 wird in einer 1-Buten-Abtrennung 14 abdestilliert. Dabei wird sogenanntes "Raffinat III" 15 erhalten.

Raffinat III 15 enthält als Wertstoff im Wesentlichen nur noch die beiden 2-Butene, die in einer Oligomerisierung 16 zu C₈-Olefinen umgesetzt werden. Das Oligomerisat 17 wird destillativ getrennt und anschließend per Hydroformylierung und Hydrierung zu Weichmacheralkoholen verarbeitet (nicht dargestellt).

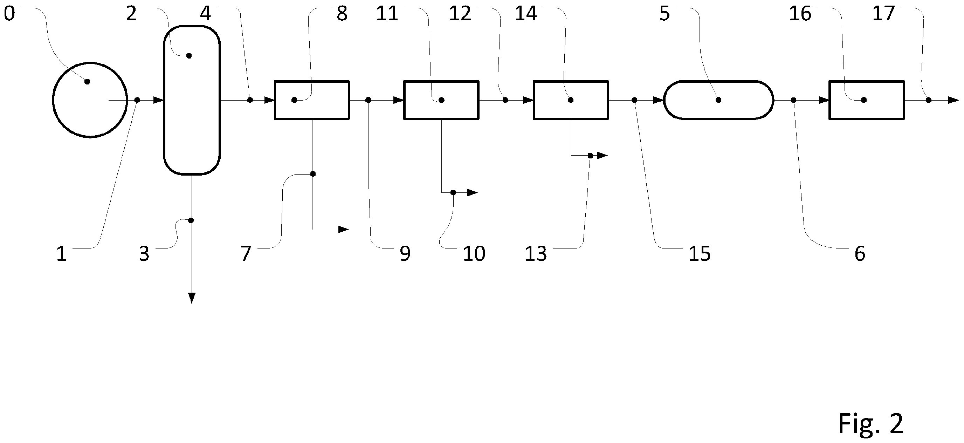

Figur 2 zeigt eine Variante eines C₄-Stranges, bei welchem das Reinigungsbett 5 unmittelbar vor der Oligomerisierung 17 angeordnet ist. Dies bietet sich insbesondere dann an, wenn als Vorreinigungsstufe 2 eine Thioveretherung eingesetzt wird, die in Gegenwart von Wasserstoff arbeitet. Der Wasserstoff wird teilweise auch noch hinter der Butadien-Abtrennung 8 benötigt, um nicht abgetrenntes Butadien selektiv zu hydrieren. Da spätestens in der Isobuten-Abtrennung 11 oder in der 1-Buten-Abtrennung 14 der Wasserstoff aus dem C₄-Strang ausgetragen wird, findet dann die Feinentschwefelung im Reinigungsbett 5 in Abwesenheit von Wasserstoff statt.

Alternativ könnte das Reinigungsbett 5 auch mit Raffinat I 9 beschickt werden. Es würde dann hinter der Butadien-Abtrennung 8 und vor der Isobuten-Abtrennung 11 angeordnet werden (nicht gezeichnet). Dies ist insbesondere dann vorteilhaft, wenn als Rohstoffgemisch 1 Crack C4 verwendet wird, welches gemäß der Spezifikation B große Mengen an 1.3-Butadien enthält. 1.3-Butadien dürfte das Sorptionsmittel zu rasch desaktivieren. Deswegen sollte das Reinigungsbett möglichst mit einem um Butadien abgereichertem Kohlenwasserstoffgemisch beaufschlagt werden, also zumindest mit Raffinat I oder mit FCC C4.

### Beispiele

### Erster Versuch: Entfernung von Ethanthiol gemäß der Erfindung

Als Sorptionsmittel wird ein von der Clariant AG erworbener Feststoff eingesetzt, der als Methanol-Katalysator verwendbar ist. Das Sorptionsmittel enthält ca. 42 Gew-% CuO, ca. 44 Gew-% ZnO, ca. 12 Gew- % Al₂O₃ und ca. 2 Gew-% Graphit und liegt in Form von Tabletten (5 x 3 mm) vor. Die spezifische Kupferoxidoberfläche beträgt, gemessen mittels Stickstoff-Sorption, 100m² pro g Kupferoxidgehalt.

In zwei Reaktionsrohre mit 1 cm Durchmesser wird je 120 g Sorptionsmittel gefüllt. Die Schüttdichte beträgt ca. 1.2 kg/dm³. Die gefüllten Rohre werden in Reihe geschaltet, wobei zwischen den Rohren (Austrag 1) und am Ende (Austrag 2) jeweils ein Probenahmeventil angebracht ist. Die Reinigungsbetten werden durch Beheizung der Rohrwände auf eine Temperatur von 80° C gebracht und bei einem Druck von 24 bar mit einem flüssigen Gemisch durchströmt, das ca. 33 Gew-% 1-Buten, ca. 23 Gew-% trans-2-Buten, ca. Gew-15 % cis-2-Buten und ca. 27 Gew-% n-Butan enthält. Als Verunreinigung enthält das Material im Mittel 5.4 mg/kg an Schwefel überwiegend in Form von Ethanthiol. Die Belastung der Reinigungsbetten beträgt 600 g/h, der Schwefeleintrag also etwa 3.2 mg/h.

Der Schwefel wird ausweislich der Analysen zunächst bereits im ersten Reinigungsbett nahezu quantitativ aus dem Gemisch entfernt. Ab einer Betriebszeit von 480 Stunden steigt der Schwefelgehalt am Austrag 1 schnell an. Dieser scharfe Durchbruch entspricht einer arretierten Schwefelmenge von etwa 1.7 g oder einer Schwefelaufnahme des Reinigungsbett von etwa 1.4 Gew.-%. Der Durchbruch nach dem zweiten Reinigungsbett (Austrag 2) erfolgt nach etwa 1200 Stunden. Die Reinigungsbetten haben zu diesem Zeitpunkt insgesamt ca. 3.9 g Schwefel aufgenommen, entsprechend einer mittleren Aufnahme von 1.7 Gew.-% bezogen auf das frisch eingefüllte Sorptionsmittel.

Die Austragswerte der einzelnen C₄-Komponenten blieben gegenüber den entsprechenden Zulaufwerten während der gesamten Versuchszeit unverändert.

Nach dem Ende dieses Versuchs werden die Betten mit Stickstoff gespült. Das Sorptionsmittel kann unversehrt und mit hinreichender Festigkeit entnommen werden.

Die Ergebnisse des Versuchs sind in Tabelle 1 niedergelegt.

**Tabelle 1: Ergebnisse aus Versuch 1**

| Mittlerer S-Gehalt [Gew.-%] Zulauf | Mittlerer S-Gehalt [Gew.-%] Austrag 1 bis 480 h | Mittlerer S-Gehalt [Gew.-%] Austrag 2 bis 1200 h | Mittlere Abnahme S [Gew.-%] in Austrag 2 gegenüber Zulauf bis 1200 h |
|---|---|---|---|
| 0.00054 | 0.00003 | 0.00002 | 96 |

### Zweiter Versuch: Entfernung von Methanthiol gemäß der Erfindung

Das verwendete Sorptionsmittel und der Versuchsaufbau entsprechen dem ersten Versuch.

Analog Versuch 1 wird als Verunreinigung 5 mg/kg an Schwefel überwiegend in Form von Methanthiol zugeführt. Die Belastung der beiden Reinigungsbetten, deren Füllmenge jeweils 28 g beträgt, liegt bei 380 g/h, der Schwefeleintrag also etwa 1.9 mg/h. Die Kontakttemperatur wurde auf 100°C eingestellt.

Der Schwefel wird ausweislich der Analysen zunächst bereits im ersten Reinigungsbett nahezu quantitativ aus dem Gemisch entfernt. Ab einer Betriebszeit von etwa 410 Stunden steigt der Schwefelgehalt am Austrag 1 an. Dieser scharfe Durchbruch entspricht einer arretierten Schwefelmenge von etwa 0.5 g oder einer Schwefelaufnahme des Sorptionsmittels von etwa 1.9 Gew.-%. Der Durchbruch nach dem zweiten Reinigungsbett (Austrag 2) erfolgt nach etwa 720 Stunden. Die Reinigungsbetten haben zu diesem Zeitpunkt insgesamt ca. 1.9 g Schwefel aufgenommen, entsprechend einer mittleren Aufnahme von 1.7 Gew.-% bezogen auf das frisch eingefüllte Sorptionsmittel.

Die Austragswerte der einzelnen C₄-Komponenten blieben gegenüber den entsprechenden Zulaufwerten während der gesamten Versuchszeit unverändert.

Nach dem Ende dieses Versuchs werden die Betten mit Stickstoff gespült. Das Sorptionsmittel kann unversehrt und mit hinreichender Festigkeit entnommen werden.

Die Versuchsergebnisse sind in Tabelle 2 dargestellt.

**Tabelle 2: Ergebnisse aus Versuch 2**

| Mittlerer S-Gehalt [Gew.-%] Zulauf | Mittlerer S-Gehalt [Gew.-%] Austrag 1 bis 410 h | Mittlerer S-Gehalt [Gew.-%] Austrag 2 bis 720 h | Mittlere Abnahme S [Gew.-%] in Austrag 2 gegenüber Zulauf bis 720 h |
|---|---|---|---|
| 0.00044 | 0.00004 | 0.00004 | 91 |

### Dritter Versuch: Entfernung von Diethyldisulfid gemäß der Erfindung

Das verwendete Sorptionsmittel und der Versuchsaufbau entsprechen dem ersten und zweiten Versuch.

Analog Versuch 1 wird als Verunreinigung 1 mg/kg an Schwefel überwiegend in Form von Diethyldisulfid zugeführt. Die Belastung der Reinigungsbetten, die jeweils 28 g des Sorptionsmittels enthalten, beträgt 360 g/h, der Schwefeleintrag also etwa 0.4 mg/h. Die Betriebstemperatur beträgt 100°C.

Der Schwefel wird ausweislich der Analysen zunächst bereits im ersten Reinigungsbett nahezu quantitativ aus dem Gemisch entfernt. Ab einer Betriebszeit von 600 Stunden steigt der Schwefelgehalt am Austrag 1 schnell an. Dieser scharfe Durchbruch entspricht einer arretierten Schwefelmenge von etwa 0.3 g oder einer Schwefelaufnahme des Sorptionsmittels von etwa 1.2Gew.-%. Der Durchbruch nach dem zweiten Reinigungsbett (Austrag 2) erfolgt nach etwa 1080 Stunden. Die Reinigungsbetten haben zu diesem Zeitpunkt insgesamt ca. 0.6 g Schwefel aufgenommen, entsprechend einer mittleren Aufnahme von 1.2 Gew.-% bezogen auf das frisch eingefüllte Sorptionsmittel.

Die Austragswerte der einzelnen C₄-Komponenten blieben gegenüber den entsprechenden Zulaufwerten während der gesamten Versuchszeit unverändert.

Nach dem Ende dieses Versuchs werden die Betten mit Stickstoff gespült. Das Sorptionsmittel kann unversehrt und mit hinreichender Festigkeit entnommen werden.

Die Versuchsergebnisse sind in Tabelle 3 dargestellt.

**Tabelle 3: Ergebnisse aus Versuch 3**

| Mittlerer S-Gehalt [Gew.-%] Zulauf | Mittlerer S-Gehalt [Gew.-%] Austrag 1 bis 600 h | Mittlerer S-Gehalt [Gew.-%] Austrag 2 bis 1080 h | Mittlere Abnahme S [Gew.-%] in Austrag 2 gegenüber Zulauf bis 1080 h |
|---|---|---|---|
| 0.00010 | 0.00001 | 0.00001 | 90 |

### Vierter Versuch: Entfernung von Dimethyldisulfid mit Hilfe von Zeolithen (nicht erfindungsgemäß)

Es wird ein Sorptionsmittel nach EP0354316 hergestellt. Es basiert auf einem Zeolithen des Typs X basiert und enthält lediglich 10 Gew.-% Cu. Die zwei mit jeweils 50 g des Materials gefüllten Rohre werden in Reihe geschaltet, wobei zwischen den Reinigungsbetten (Austrag 1) und am Ende (Austrag 2) jeweils ein Probenahmeventil angebracht ist. Die Betten werden durch Beheizung der Rohrwände auf eine Temperatur von 120° C gebracht und bei einem Druck von 30 bar mit einem flüssigen Gemisch durchströmt, das ca. 33 Gew-% 1-Buten, ca. 23 Gew-% trans-2-Buten, ca. Gew-15 % cis-2-Buten und ca. 27 Gew-% n-Butan enthält. Als Verunreinigung enthält das Material im Mittel 2.0 mg/kg an Schwefel überwiegend in Form von Dimethyldisulfid. Die Belastung der Reinigungsbetten beträgt 375 g/h, der Schwefeleintrag also etwa 0.75 mg/h.

Der Schwefel wird ausweislich der Analysen zunächst bereits im ersten Reaktor nahezu quantitativ aus dem Gemisch entfernt. Ab einer Betriebszeit von 48 Stunden steigt der Schwefelgehalt am Austrag 1 aber schnell an. Dieser scharfe Durchbruch entspricht einer adsorbierten Schwefelmenge von nur etwa 0.036 g oder einer Schwefelaufnahme des Sorptionsmittels von etwa 0.036 Gew.-%. Der Durchbruch nach dem zweiten Reinigungsbett (Austrag 2) erfolgt nach etwa 96 Stunden. Die Reinigungsbetten haben zu diesem Zeitpunkt insgesamt ca. 0.07 g Schwefel aufgenommen, entsprechend einer mittleren Aufnahme von 0.07 Gew.-% bezogen auf das frisch eingefüllte Sorptionsmittel.

Mit dem nicht erfindungsgemäßen Material ist demnach nur für sehr kurze Zeit eine deutliche Entschwefelung zu erreichen, wobei der Materialeinsatz in keinem Verhältnis zur Reinigungsleistung steht.

Die Ergebnisse sind in Tabelle 4 dargestellt.

**Tabelle 4: Ergebnisse aus Versuch 4**

| Mittlerer S-Gehalt [Gew.-%] Zulauf | Mittlerer S-Gehalt [Gew.-%] Austrag 1 bis 48 h | Mittlerer S-Gehalt [Gew.-%] Austrag 2 bis 96 h | Mittlere Abnahme S [Gew.-%] in Austrag 2 gegenüber Zulauf bis 96 h |
|---|---|---|---|
| 0.00020 | 0.000005 | 0.000005 | 97 |

**FAZIT:** Die Versuche belegen, dass das erfindungsgemäß eingesetzte Sorptionsmittel die folgenden Eigenschaften aufweist:
- Es bindet den Schwefel aus Schwefelverbindungen nahezu vollständig;
- Es benötigt keine Aktivierung im Wasserstoffstrom und auch sonst keine zusätzlichen Betriebsstoffe;
- Es benötigt keine periodischen Reinigungs- und Desorptionsströme, da es sich um ein irreversibles Sorptionsmittel handelt;
- Es kann in einem einfachen Behälter untergebracht werden, der einfach vom dem Gemisch durchströmt wird, vorzugsweise bei leicht erhöhter Temperatur, wie sie typischerweise oft ohnehin für die Speisung von nachfolgenden Reaktoren erforderlich ist;
- Es verursacht praktisch keine Nebenreaktionen der Olefine wie Oligomerisierung, Isomerisierung und Hydrierung und damit auch keine Verluste;
- Es setzt keinerlei Stoffe in Konzentrationen frei, die irgendeinen Einfluss auf die nachfolgenden Verarbeitungsstufen haben;
- Es ist angesichts der bei typischen Schwefelkonzentrationen unter 5 ppmw und eine Kapazität von mindestens 1 Gew.-% Schwefel langen Lebensdauer sehr kostengünstig im Betrieb, auch wenn es nicht direkt regeneriert werden kann, sondern nach Erschöpfung der Kapazität nur noch einer rohstofflichen Verwertung zugeführt werden kann;
- Es lässt sich gefahrlos handhaben und entsorgen, da es weder als kanzerogen eingestuft ist noch pyrophore Eigenschaften zeigt.

### Bezugszeichenliste

- 0: Rohstoffquelle
- 1: Rohstoffgemisch
- 2: Vorreinigungsstufe
- 3: schwefelhaltige Bestandteile
- 4: verunreinigtes Kohlenwasserstoffgemisch
- 5: Reinigungsbett
- 6: gereinigtes Kohlenwasserstoffgemisch
- 7: Butadien
- 8: Butadien-Abtrennung
- 9: Raffinat I
- 10: Isobuten
- 11: Isobuten-Abtrennung (MTBE-Synthese/MTBE-Spaltung)
- 12: Raffinat II
- 13: 1-Buten
- 14: 1-Buten-Abtrennung
- 15: Raffinat III
- 16: Oligomerisierung
- 17: Oligomerisat

## Patentansprüche

1. Verfahren zur Reinigung von Kohlenwasserstoffgemischen, bei welchem ein verunreinigtes Kohlenwasserstoffgemisch enthaltend Kohlenwasserstoffe mit drei bis acht Kohlenstoffatomen durch Kontaktieren mit einem festen Sorptionsmittel zumindest teilweise von Verunreinigungen befreit wird, wobei sich das Kohlenwasserstoffgemisch während des Kontakts mit dem Sorptionsmittel ausschließlich im flüssigen Zustand befindet,
wobei das Sorptionsmittel die folgende, sich zu 100 Gew.-% ergänzende Zusammensetzung aufweist:
• Kupferoxid: 10 bis 60 Gew.-% (gerechnet als CuO);
• Zinkoxid: 10 bis 60 Gew.-%, (gerechnet als ZnO);
• Aluminiumoxid: 10 bis 30 Gew.-% (gerechnet als Al₂O₃);
• sonstige Stoffe: 0 bis 5 Gew.-%;
und wobei
das verunreinigte Kohlenwasserstoffgemisch mindestens eine Verunreinigung aus einer der folgenden Substanzklassen enthält:
a) Thiole mit der allgemeinen Formel R-SH
wobei R ein Alkyl-, Aryl-, Cycloalkyl- oder ein Alkenyl-Rest sein kann, wobei es sich bei R insbesondere ein Methyl-, Ethyl-, Propyl-, Butyl-, Phenyl-, Cyclohexyl- oder Butenyl-Rest handelt.
b) Disulfide mit der allgemeinen Formel R-S-S-R',
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
c) Sulfide mit der allgemeinen Formel R-S-R'
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
d) Substitutierte oder unsubstituierte schwefelhaltige Heterocyclen, insbesondere Thiophene und/oder Thiolane,
**dadurch gekennzeichnet,**
**dass** es sich bei den zu entfernenden Verunreinigungen um organische Schwefelverbindungen aus den Substanzklassen a), b), c) und d) handelt,
**dass** der Kontakt unter den folgenden Bedingungen erfolgt:
• Temperatur zwischen 30°C und 120°C;
• Druck zwischen 0.5 und 3.5 MPa;
• Raum/Zeit-Belastung (weight hour space velocity - WHSV) zwischen 0.5 h⁻¹ und 7 h⁻¹
und **dass** das verunreinigte Kohlenwasserstoffgemisch eine der folgenden, sich jeweils zu 100 Gew.-% ergänzende Spezifikationen A, B, C oder D erfüllt, wobei sich die angegebenen Gewichtsanteile jeweils auf das Gesamtgewicht des verunreinigten Kohlenwasserstoffgemisches beziehen:
Spezifikation A:
• Isobutan 20 bis 40 Gew.-%, bevorzugt 30 bis 37 Gew.-%;
• n-Butan 5 bis 18 Gew.-%, bevorzugt 8 bis 10 Gew.-%;
• 1-Buten 5 bis 15 Gew.-%, bevorzugt 12 bis 14 Gew.-%;
• Isobuten 12 bis 25 Gew.-%, bevorzugt 15 bis 20 Gew.-%;
• 2-Butene 9 bis 40 Gew.-%, bevorzugt 20 bis 30 Gew.-%;
• 1.3-Butadien 0 bis 3 Gew.-%, bevorzugt 0.5 bis 0.8 Gew.-%;
• Wasser 0 bis 1 Gew.-%, bevorzugt weniger als 0.1 Gew.-%;
• Verunreinigungen, insbesondere Schwefel enthaltende Kohlenwasserstoffe von weniger als 0.5 Gew.-%, bevorzugt kleiner 0.2 Gew.-%;
Spezifikation B:
• Isobutan 0.6 bis 8 Gew.-%, bevorzugt 1 bis 7 Gew.-%;
• n-Butan 0.5 bis 8 Gew.-%, bevorzugt 4 bis 7 Gew.-%;
• 1-Buten 9 bis 25 Gew.-%, bevorzugt 10 bis 20 Gew.-%;
• Isobuten 10 bis 35 Gew.-%, bevorzugt 20 bis 30 Gew.-%;
• 2-Butene 3 bis 15 Gew.-%, bevorzugt 5 bis 10 Gew.-%;
• 1.3-Butadien 25 bis 70 Gew.-%, bevorzugt 40 bis 50 Gew.-%;
• Wasser 0 bis 1 Gew.-%, bevorzugt weniger als 0.5 Gew.-%;
• Verunreinigungen, insbesondere Schwefel enthaltene Kohlenwasserstoffe weniger als 0.5 Gew.-%, bevorzugt kleiner 0.2 Gew.-%;
Spezifikation C:
• Isobutan 0.6 bis 8 Gew.-%, bevorzugt 1 bis 7 Gew.-%;
• n-Butan 0.5 bis 15 Gew.-%, bevorzugt 4 bis 13 Gew.-%;
• 1-Buten 9 bis 40 Gew.-%, bevorzugt 10 bis 35 Gew.-%;
• Isobuten 10 bis 55 Gew.-%, bevorzugt 20 bis 50 Gew.-%;
• 2-Butene 3 bis 25 Gew.-%, bevorzugt 5 bis 20 Gew.-%;
• 1.3-Butadien 0 bis 1 Gew.-%, bevorzugt weniger als 0.8 Gew.-%;
• Wasser 0 bis 1 Gew.-%, bevorzugt weniger als 0.5 Gew.-%;
• Verunreinigungen, insbesondere Schwefel enthaltene Kohlenwasserstoffe weniger als 0.5 Gew.-%, bevorzugt kleiner 0.2 Gew.-%;
Spezifikation D:
• n-Butan 10 bis 30 Gew.-%, bevorzugt 25 bis 30 Gew.-%;
• 1-Buten 0.2 bis 45 Gew.-%, bevorzugt 5 bis 30 Gew.-%;
• 2-Butene 35 bis 85 Gew.-%, bevorzugt 50 bis 75 Gew.-%;
• Wasser 0 bis 1 Gew.-%, bevorzugt weniger als 0.1 Gew.-%;
• Verunreinigungen, insbesondere Schwefel enthaltene Kohlenwasserstoffe weniger als 0.5 Gew.-%, bevorzugt kleiner 0.1 Gew.-%;
wobei das Verfahren zur Reinigung von Kohlenwasserstoffgemischen bei einem Wasserstoffgehalt von unter 1 Gew.-ppm bezogen auf die Gesamtmasse des verunreinigten Kohlenwasserstoffgemisches durchgeführt wird.

2. Verfahren nach Anspruch 1,
**dadurch gekennzeichnet,**
**dass** das Sorptionsmittel die folgende, sich zu 100 Gew.-% ergänzende Zusammensetzung aufweist:
• Kupferoxid: 30 bis 45 Gew.-% (gerechnet als CuO);
• Zinkoxid: 30 bis 50 Gew.-%, (gerechnet als ZnO);
• Aluminiumoxid: 10 bis 15 Gew.-% (gerechnet als Al₂O₃);
• weitere Metalloxide: 0 bis 2 Gew.-%;
• Graphit: 0 bis 3 Gew.-%;
• andere Stoffe: 0 bis 1 Gew.-%.

3. Verfahren nach einem der vorhergehenden Ansprüche,
**dadurch gekennzeichnet,**
**dass** der Gewichtsanteil der Verunreinigungen in dem verunreinigten Kohlenwasserstoffgemisch bezogen auf dessen Gesamtgewicht kleiner ist als 0.2 Gew.-%; besonders bevorzugt, dass er unter 100 Gew.-ppm liegt und ganz besonders bevorzugt, dass er unter 10 Gew.-ppm liegt.

4. Verfahren nach einem der vorhergehenden Ansprüche,
**dadurch gekennzeichnet,**
**dass** das verunreinigte Kohlenwasserstoffgemisch aus einer Vorreinigungsstufe erhalten wird, welche ein stärker verunreinigtes Rohstoffgemisch unter Erhalt des verunreinigten Kohlenwasserstoffgemisches vorreinigt.

5. Verfahren nach Anspruch 4,
**dadurch gekennzeichnet,**
**dass** das Sorptionsmittel irreversibel eingesetzt wird.

6. Verfahren nach einem der vorhergehenden Ansprüche,
**dadurch gekennzeichnet,**
**dass** das zumindest teilweise von Verunreinigungen befreite Kohlenwasserstoffgemisch zumindest einem der nachfolgend aufgezählten Aufarbeitungsschritte unterworfen wird:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.

## Claims

1. Process for purifying hydrocarbon mixtures, in which a contaminated hydrocarbon mixture comprising hydrocarbons having three to eight carbon atoms is at least partly freed of contaminants by contacting it with a solid sorbent, the hydrocarbon mixture being exclusively in the liquid state during the contact with the sorbent,
wherein the sorbent has the following composition that adds up to 100% by weight:
• copper oxide: 10% to 60% by weight (calculated as CuO);
• zinc oxide: 10% to 60% by weight (calculated as ZnO);
• aluminium oxide: 10% to 30% by weight (calculated as Al₂O₃) ;
• other substances: 0% to 5% by weight;
and wherein
the contaminated hydrocarbon mixture contains at least one impurity from one of the following substance classes:
a) thiols having the general formula R-SH
where R may be an alkyl, aryl, cycloalkyl or alkenyl radical, where R is especially a methyl, ethyl, propyl, butyl, phenyl, cyclohexyl or butenyl radical;
b) disulphides having the general formula R-S-S-R'
where R and R' may be identical or different alkyl, aryl, cycloalkyl or alkenyl radicals, where R and R' are especially methyl, ethyl, propyl, butyl, phenyl, cyclohexyl or butenyl radicals;
c) sulphides having the general formula R-S-R'
where R and R' may be identical or different alkyl, aryl, cycloalkyl or alkenyl radicals, where R and R' are especially methyl, ethyl, propyl, butyl, phenyl, cyclohexyl or butenyl radicals;
d) substituted or unsubstituted sulphur-containing heterocycles, especially thiophenes and/or thiolanes,
**characterized in that**
the impurities to be removed are organic sulphur compounds from the substance claims a), b), c) and d),
**in that** the contact is effected under the following conditions:
• temperature between 30°C and 120°C;
• pressure between 0.5 and 3.5 MPa;
• space-time yield (weight hourly space velocity - WHSV) between 0.5 h⁻¹ and 7 h⁻¹,
and **in that** the contaminated hydrocarbon mixture fulfils one of the following specifications A, B, C and D, each of which adds up to 100% by weight, the stated proportions by weight each being based on the total weight of the contaminated hydrocarbon mixture:
Specification A:
• isobutane 20% to 40% by weight, preferably 30% to 37% by weight;
• n-butane 5% to 18% by weight, preferably 8% to 10% by weight;
• 1-butene 5% to 15% by weight, preferably 12% to 14% by weight;
• isobutene 12% to 25% by weight, preferably 15% to 20% by weight;
• 2-butenes 9% to 40% by weight, preferably 20% to 30% by weight;
• 1,3-butadiene 0% to 3% by weight, preferably 0.5% to 0.8% by weight;
• water 0% to 1% by weight, preferably less than 0.1% by weight;
• contaminants, especially sulphur-containing hydrocarbons, less than 0.5% by weight, preferably less than 0.2% by weight;
Specification B:
• isobutane 0.6% to 8% by weight, preferably 1% to 7% by weight;
• n-butane 0.5% to 8% by weight, preferably 4% to 7% by weight;
• 1-butene 9% to 25% by weight, preferably 10% to 20% by weight;
• isobutene 10% to 35% by weight, preferably 20% to 30% by weight;
• 2-butenes 3% to 15% by weight, preferably 5% to 10% by weight;
• 1,3-butadiene 25% to 70% by weight, preferably 40% to 50% by weight;
• water 0% to 1% by weight, preferably less than 0.5% by weight;
• contaminants, especially sulphur-containing hydrocarbons, less than
0.5% by weight, preferably less than 0.2% by weight;
Specification C:
• isobutane 0.6% to 8% by weight, preferably 1% to 7% by weight;
• n-butane 0.5% to 15% by weight, preferably 4% to 13% by weight;
• 1-butene 9% to 40% by weight, preferably 10% to 35% by weight;
• isobutene 10% to 55% by weight, preferably 20% to 50% by weight;
• 2-butenes 3% to 25% by weight, preferably 5% to 20% by weight;
• 1,3-butadiene 0% to 1% by weight, preferably less than 0.8% by weight;
• water 0% to 1% by weight, preferably less than 0.5% by weight;
• contaminants, especially sulphur-containing hydrocarbons, less than
0.5% by weight, preferably less than 0.2% by weight;
Specification D:
• n-butane 10% to 30% by weight, preferably 25% to 30% by weight;
• 1-butene 0.2% to 45% by weight, preferably 5% to 30% by weight;
• 2-butenes 35% to 85% by weight, preferably 50% to 75% by weight;
• water 0% to 1% by weight, preferably less than 0.1% by weight;
• contaminants, especially sulphur-containing hydrocarbons, less than
0.5% by weight, preferably less than 0.1% by weight;
wherein the process for purification of hydrocarbon mixtures is conducted at a hydrogen content of below 1 ppm by weight, based on the total mass of the contaminated hydrocarbon mixture.

2. Process according to Claim 1,
**characterized in that**
the sorbent has the following composition that adds up to 100% by weight:
• copper oxide: 30% to 45% by weight (calculated as CuO);
• zinc oxide: 30% to 50% by weight (calculated as ZnO);
• aluminium oxide: 10% to 15% by weight (calculated as Al₂O₃) ;
• further metal oxides: 0% to 2% by weight;
• graphite: 0% to 3% by weight;
• other substances: 0% to 1% by weight.

3. Process according to any of the preceding claims,
**characterized in that**
the proportion by weight of the contaminants in the contaminated hydrocarbon mixture, based on the total weight thereof, is less than 0.2% by weight, more preferably below 100 ppm by weight and most preferably below 10 ppm by weight.

4. Process according to any of the preceding claims,
**characterized in that**
the contaminated hydrocarbon mixture is obtained from a pre-purification stage which pre-purifies a more highly contaminated raw material mixture to obtain the contaminated hydrocarbon mixture.

5. Process according to Claim 4,
**characterized in that**
the sorbent is used irreversibly.

6. Process according to any of the preceding claims,
**characterized in that**
the hydrocarbon mixture which has been at least partly freed of contaminants is subjected to at least one of the workup steps enumerated below:
a) extraction of 1,3-butadiene present in the hydrocarbon mixture;
b) selective hydrogenation of diolefins and/or acetylenes present in the hydrocarbon mixture to olefins;
c) oligomerization of olefins present in the hydrocarbon mixture to corresponding oligomers;
d) distillative removal of 1-butene and/or isobutane present in the hydrocarbon mixture, especially with the purpose of obtaining 1-butene and/or isobutane in high purity;
e) removal of isobutene present in the hydrocarbon mixture by conversion of the isobutene with water to tert-butanol and/or with methanol to methyl tert-butyl ether;
f) dehydrogenation of butanes present in the hydrocarbon mixture to butenes;
g) oxidative dehydrogenation of butenes present in the hydrocarbon mixture to butadiene;
h) alkylation of n-butene present in the hydrocarbon mixture with isobutane likewise present;
i) oxidation of hydrocarbons having four carbon atoms present in the hydrocarbon mixture for preparation of maleic anhydride.

## Revendications

1. Procédé de purification de mélanges d'hydrocarbures, selon lequel un mélange d'hydrocarbures contaminé contenant des hydrocarbures de trois à huit atomes de carbone est au moins partiellement débarrassé des impuretés par mise en contact avec un sorbant solide, le mélange d'hydrocarbures se trouvant exclusivement à l'état liquide pendant la mise en contact avec le sorbant,
le sorbant présentant la composition suivante, dont la somme est de 100 % en poids :
- oxyde de cuivre : 10 à 60 % en poids (calculé en tant que CuO) ;
- oxyde de zinc : 10 à 60 % en poids (calculé en tant que ZnO) ;
- oxyde d'aluminium : 10 à 30 % en poids (calculé en tant qu'Al₂O₃) ;
- autres substances : 0 à 5 % en poids ;
et
le mélange d'hydrocarbures contaminé contenant au moins une impureté d'une des classes de substances suivantes :
a) les thiols de formule générale R-SH,
R pouvant être un radical alkyle, aryle, cycloalkyle ou alcényle, R étant notamment un radical méthyle, éthyle, propyle, butyle, phényle, cyclohexyle ou butényle,
b) les disulfures de formule générale R-S-S-R',
R et R' pouvant être des radicaux alkyle, aryle, cycloalkyle ou alcényle identiques ou différents, R et R' étant notamment des radicaux méthyle, éthyle, propyle, butyle, phényle, cyclohexyle ou butényle ;
c) les sulfures de formule générale R-S-R',
R et R' pouvant être des radicaux alkyle, aryle, cycloalkyle ou alcényle identiques ou différents, R et R' étant notamment des radicaux méthyle, éthyle, propyle, butyle, phényle, cyclohexyle ou butényle ;
d) les hétérocycles contenant du soufre substitués ou non substitués, notamment les thiophènes et/ou les thiolanes,
**caractérisé en ce que**
les impuretés à éliminer consistent en des composés de soufre organiques des classes de substances a) , b) , c) et d),
**en ce que** la mise en contact a lieu dans les conditions suivantes :
- température comprise entre 30 °C et 120 °C ;
- pression comprise entre 0,5 et 3,5 MPa ;
- chargement espace/temps (weight hour space velocity
- WHSV) compris entre 0,5 h⁻¹ et 7 h⁻¹,
et **en ce que** le mélange d'hydrocarbures contaminé satisfait une des spécifications A, B, C ou D suivante, dont la somme est à chaque fois de 100 % en poids, les proportions en poids indiquées se rapportant chacune au poids total du mélange d'hydrocarbures contaminé :
spécification A :
- isobutane 20 à 40 % en poids, de préférence 30 à 37 % en poids ;
- n-butane 5 à 18 % en poids, de préférence 8 à 10 % en poids ;
- 1-butène 5 à 15 % en poids, de préférence 12 à 14 % en poids ;
- isobutène 12 à 25 % en poids, de préférence 15 à 20 % en poids ;
- 2-butènes 9 à 40 % en poids, de préférence 20 à 30 % en poids ;
- 1,3-butadiène 0 à 3 % en poids, de préférence 0,5 à 0,8 % en poids ;
- eau 0 à 1 % en poids, de préférence moins de 0,1 % en poids ;
- impuretés, notamment hydrocarbures contenant du soufre, moins de 0,5 % en poids, de préférence moins de 0,2 % en poids ;
spécification B :
- isobutane 0,6 à 8 % en poids, de préférence 1 à 7 % en poids ;
- n-butane 0,5 à 8 % en poids, de préférence 4 à 7 % en poids ;
- 1-butène 9 à 25 % en poids, de préférence 10 à 20 % en poids ;
- isobutène 10 à 35 % en poids, de préférence 20 à 30 % en poids ;
- 2-butènes 3 à 15 % en poids, de préférence 5 à 10 % en poids ;
- 1,3-butadiène 25 à 70 % en poids, de préférence 40 à 50 % en poids ;
- eau 0 à 1 % en poids, de préférence moins de 0,5 % en poids ;
- impuretés, notamment hydrocarbures contenant du soufre, moins de 0,5 % en poids, de préférence moins de 0,2 % en poids ;
spécification C :
- isobutane 0,6 à 8 % en poids, de préférence 1 à 7 % en poids ;
- n-butane 0,5 à 15 % en poids, de préférence 4 à 13 % en poids ;
- 1-butène 9 à 40 % en poids, de préférence 10 à 35 % en poids ;
- isobutène 10 à 55 % en poids, de préférence 20 à 50 % en poids ;
- 2-butènes 3 à 25 % en poids, de préférence 5 à 20 % en poids ;
- 1,3-butadiène 0 à 1 % en poids, de préférence moins de 0,8 % en poids ;
- eau 0 à 1 % en poids, de préférence moins de 0,5 % en poids ;
- impuretés, notamment hydrocarbures contenant du soufre, moins de 0,5 % en poids, de préférence moins de 0,2 % en poids ;
spécification D :
- n-butane 10 à 30 % en poids, de préférence 25 à 30 % en poids ;
- 1-butène 0,2 à 45 % en poids, de préférence 5 à 30 % en poids ;
- 2-butènes 35 à 85 % en poids, de préférence 50 à 75 % en poids ;
- eau 0 à 1 % en poids, de préférence moins de 0,1 % en poids ;
- impuretés, notamment hydrocarbures contenant du soufre, moins de 0,5 % en poids, de préférence moins de 0,1 % en poids ;
le procédé de purification de mélanges d'hydrocarbures étant réalisé à une teneur en hydrogène inférieure à 1 ppm en poids, par rapport à la masse totale du mélange d'hydrocarbures contaminé.

2. Procédé selon la revendication 1, **caractérisé en ce que** le sorbant présente la composition suivante, dont la somme est de 100 % en poids :
- oxyde de cuivre : 30 à 45 % en poids (calculé en tant que CuO) ;
- oxyde de zinc : 30 à 50 % en poids (calculé en tant que ZnO) ;
- oxyde d'aluminium : 10 à 15 % en poids (calculé en tant qu'Al₂O₃) ;
- autres oxydes métalliques : 0 à 2 % en poids ;
- graphite : 0 à 3 % en poids ;
- autres substances : 0 à 1 % en poids.

3. Procédé selon l'une quelconque des revendications précédentes, **caractérisé en ce que** la proportion en poids des impuretés dans le mélange d'hydrocarbures contaminé, par rapport à son poids total, est inférieure à 0,2 % en poids, de manière particulièrement préférée inférieure à 100 ppm en poids et de manière tout particulièrement préférée inférieure à 10 ppm en poids.

4. Procédé selon l'une quelconque des revendications précédentes, **caractérisé en ce que** le mélange d'hydrocarbures contaminé est obtenu à partir d'une étape de purification préliminaire, qui purifie de manière préliminaire un mélange de matières premières plus fortement contaminé pour obtenir le mélange d'hydrocarbures contaminé.

5. Procédé selon la revendication 4, **caractérisé en ce que** le sorbant est utilisé de manière irréversible.

6. Procédé selon l'une quelconque des revendications précédentes, **caractérisé en ce que** le mélange d'hydrocarbures au moins partiellement débarrassé des impuretés est soumis à au moins une des étapes de traitement énumérées ci-après :
a) une extraction du 1,3-butadiène contenu dans le mélange d'hydrocarbures ;
b) une hydrogénation sélective des dioléfines et/ou acétylènes contenus dans le mélange d'hydrocarbures en oléfines ;
c) une oligomérisation des oléfines contenues dans le mélange d'hydrocarbures en les oligomères correspondants ;
d) une séparation par distillation du 1-butène et/ou de l'isobutane contenus dans le mélange d'hydrocarbures, notamment dans le but d'obtenir du 1-butène et/ou de l'isobutane en une pureté plus élevée ;
e) l'élimination de l'isobutène contenu dans le mélange d'hydrocarbures par transformation de l'isobutène avec de l'eau en tert.-butanol et/ou avec du méthanol en éther de méthyle et de tert.-butyle ;
f) la déshydrogénation des butanes contenus dans le mélange d'hydrocarbures en butènes ;
g) la déshydrogénation oxydative des butènes contenus dans le mélange d'hydrocarbures en butadiène ;
h) l'alkylation du n-butène contenu dans le mélange d'hydrocarbures avec l'isobutane également contenu ;
i) l'oxydation des hydrocarbures contenant quatre atomes de carbone contenus dans le mélange d'hydrocarbures pour fabriquer de l'anhydride de l'acide maléique.
